(19) **Europäisches Patentamt**
**European Patent Office**
**Office européen des brevets**

(11) Publication number: **0 406 617 A2**

## (12) EUROPEAN PATENT APPLICATION

(21) Application number: **90111620.2**

(22) Date of filing: **20.06.90**

(51) Int. Cl.⁵: **D21C 3/00, C12S 3/00**

(30) Priority: **22.06.89 US 370286**

(43) Date of publication of application:
**09.01.91 Bulletin 91/02**

(84) Designated Contracting States:
**AT BE CH DE DK ES FR GB IT LI NL SE**

(71) Applicant: **INTERNATIONAL PAPER COMPANY**
**2 Manhattanville Road**
**Purchase New York 10577(US)**

(72) Inventor: **Olsen, William L.**
**37 Pumpkin Hill Road**
**Warwick, N.Y. 10990(US)**
Inventor: **Gallagher, Hugh P.**
**63 Main Street**
**Chester, N.Y. 10918(US)**
Inventor: **Burris, Kathleen A.**
**76 South Jackson Avenue**
**New Windsor, N.Y. 12550(US)**
Inventor: **Bhattacharjee, Shyam S.**
**22 Chatham Road**
**Monroe, N.Y. 10950(US)**
Inventor: **Slocomb, John P.**
**9 Lower Hillman Road**
**Warwick, N.Y. 10990(US)**
Inventor: **DeWitt, Doraine M.**
**15 Black Stallion Court**
**Middletown, N.Y. 10940(US)**

(74) Representative: **Reichel, Wolfgang, Dipl.-Ing.**
**et al**
**Reichel und Reichel Parkstrasse 13**
**D-6000 Frankfurt am Main 1(DE)**

(54) **Enzymatic delignification of lignocellulosic material.**

(57) A plural stage process for the enzymatic delignification of lignocellulosic materials for use in the pulp and paper industry comprising one or more ligninolytic enzyme treatment stages ard one or more xylanase treatment stages. The ligninolytic enzyme preparation may comprise lignin peroxidases, Mn(II)-dependent peroxidases, or both these enzymes.

EP 0 406 617 A2

## ENZYMATIC DELIGNIFICATION OF LIGNOCELLULOSIC MATERIAL

### TECHNICAL FIELD OF THE INVENTION

This invention relates, in a first embodiment, to a single or plural stage process for the enzymatic delignification of lignocellulosic materials with ligninolytic enzymes. In a second embodiment, this invention relates to a plural stage process for the enzymatic delignification of lignocellulosic materials with ligninolytic enzymes and with xylanases. More particularly, the invention is directed to an improved process for the enzymatic delignification of lignocellulosic materials for use in the pulp and paper industry.

### BACKGROUND OF THE INVENTION

The major components of lignocellulosic materials (e.g. wood) are cellulose, hemicellulose, and lignin. These compounds respectively comprise approximately 35-50%, 20-30% and 20-30% of the dry weight of woody plants. Cellulose is a saccharide polymer composed of D-glucose units arranged in a linear array. In woody plants, the linear cellulose molecules are arranged in densely packed fibril bundles. Hemicelluloses are linear and branched saccharide homo- and heteropolymers composed of various five and six carbon sugars. These sugars include, for example, xylose, arabinose, mannose, galactose and glucose. Hemicelluloses consisting of a homopolymer of one of these sugars would be termed, respectively, xylan, arabinan, mannan, galactan and glucan. Hemicelluloses participate in cross-linking the cellulose molecules and fibril bundles. The other major component of lignocellulosic material is lignin. Like cellulose and the hemicelluloses, lignin is a natural polymer. Lignin, however, is more complex than either cellulose or the hemicelluloses. It is composed primarily of methoxylated phenylpropane units which have been randomly linked by a variety of carbon-carbon and ether linkages, resulting in a three-dimensional matrix. This matrix encases the cellulose fibrils, imparting strength and rigidity to the composition. Thus, lignin can be considered as a kind of natural "cement" that holds together and surrounds the cellulose fibers.

Paper is basically a two-dimensional meshwork of randomly arranged cellulosic fibers linked by hydrogen bonds between the polysaccharide units. In woody plants the cellulose fibers are "cemented" into ordered parallel arrays by lignin. Therefore, to make woody plant material useful for papermaking, it must be separated into individual cellulosic fibers capable of hydrogen bonding to other cellulosic fibers. In conventional processing of woody plant material, fibers are liberated by mechanical grinding or refining, by chemical modification or removal of lignin, or by combinations of these methods. Fiber separation results in the formation of paper pulp -- a slurry or suspension of wood fibers. The deposition of these fibers into a tangled mat results in paper.

Mechanical pulping is the physical separation of woody fibers, producing so called high-yield pulp. High yield is obtained because lignin is not removed during the pulping process and so contributes its mass to the pulp.

In chemical pulping, the lignocellulosic material is treated with harsh chemical oxidants which degrade lignin. Chemical pulping is most commonly achieved with the Kraft (sulfate), sulfite, soda, and modified sulfite processes. These treatments remove the bulk of the lignin matrix, which frees the cellulose fibers and causes the formation of pulp. For example, the Kraft (sulfate) pulping process produces a pulp containing only 5-8% by weight of residual lignin, an approximately three to five fold reduction in the original lignin content.

Hybrid processes such as thermomechanical, chemithermomechanical and chemimechanical pulping have also found use.

Each of the above described pulping processes results in dark colored pulp. The color associated with paper pulp is almost exclusively due to residual lignin. The intensity of pulp color depends on both the total amount of residual lignin and on its chemical state. For example, chemical pulps, from which most lignin has been removed during pulping, are especially dark in color because the remaining lignin is extensively oxidized and modified.

The residual lignin remaining in chemical pulps is particularly refractory to removal. This difficulty has been attributed to covalent bonding of residual lignin to hemicellulose (e.g., xylan), and perhaps to cellulose. Some of these bonds may be present in wood. However, the majority are thought to be formed during the chemical pulping process. See, e.g., Matsumoto et al., "The Role of Sugars Remaining in Residual Lignin", Fourth International Symposium on Wood and Pulping Chemistry , Paris, France, April 1987, Vol. 2, pp. 305-11; Iversen et al., "The Formation of Lignin-Carbohydrate Bonds During Kraft Pulping," Fourth International Symposium on Wood and Pulping Chemistry , Paris, France, April 1987, Vol. 2, pp. 163-65; Iversen and Wannstrom, "Lignin-Carbohydrate Bonds in a Residual Lignin Isolated from Pine Kraft Pulp", Holzforschung , 40, pp. 19-22 (1986).

There are many grades of paper, ranging from high quality white paper to that used in corrugated boxes. In order to produce a desirably bright paper product, the pulp must be brightened prior to its conversion into paper. In addition, it is desirable that high quality white papers not be subject to brightness reversion (i.e., yellowing) in response to light and aging.

Pulp may be brightened in either of two ways. First, the chromophoric groups of lignin may be destroyed, without removing the bulk of the lignin. Second, the lignin (including its chromophoric groups) may be almost totally removed from the pulp. The first method is usually reserved for brightening mechanical pulps, where retention of the bulk of lignin mass is necessary to maintain high yield. The second method is usually used to brighten chemical pulps used in the production of high quality white paper. Additionally, since brightness reversion is proportional to lignin content, only pulp brightened by the second method is capable of producing permanently bright paper (i.e., paper not subject to yellowing).

The quality of brightness is commonly measured in terms of % G.E. - a measure of reflectance. The lignin content of pulp or paper products is commonly quantified in terms of kappa number. Although the dark color of pulp is due to lignin chromophores, brightness (% G.E.) is not directly proportional to lignin content (kappa number). For example, a bleaching stage which reduced the kappa number of a pulp from 25 to 15, without destroying lignin chromophores, would not significantly brighten the pulp. The kappa number must fall below about 8 to 12 before significant increases in % G.E. are observed as a result of lignin removal. Thus, extensive delignification may be required before measurable brightening is achieved.

The process of brightening pulp is referred to as bleaching. Pulp bleaching is commonly a multistage process. Brightening is not necessarily observed at each stage of a bleaching process, or even after the first several stages. It is the sum total of the bleaching stages that results in a brightened, bleached pulp. Single or plural stage processes which destroy lignin chromophores, remove lignin (delignify), or accomplish both these ends, might be termed bleaching stages.

The most commonly used bleaching processes employ chlorine or chlorine-containing compounds such as calcium hypochlorite, sodium hypochlorite and chlorine dioxide. These processes bleach pulp primarily by removing nearly all its lignin, rather than by destroying the lignin chromophores. The first stages of these processes are usually chlorination and alkaline extraction of the pulp. While these stages result in effective bleaching, they have severe drawbacks. First, the harsh nature of these treatments causes significant degradation of cellulose. The extent of this degradation may be monitored by measuring pulp viscosity. The greater the chain length of the cellulose molecules, the higher the pulp viscosity will be. Since paper is basically a tangled mat of cellulose molecules, a decrease in chain length results in a paper product with reduced strength and tear resistance. Thus, a bleaching process which minimizes the degradation of cellulose fibers would be preferable.

A more important drawback of the chlorination extraction stage is that the effluent from this stage is highly corrosive and contains a large number of chlorinated lignin breakdown products. Some of these chlorinated organics are toxic, and potentially mutagenic and/or carcinogenic. Disposal of this effluent thus presents a serious waste problem. Another drawback is that the highly corrosive chlorides in this effluent attack plant machinery and thus preclude effluent recycling for use in other stages of the paper making process. Finally, the corrosive nature of this effluent makes it hazardous to plant personnel. For these reasons, alternative bleaching processes which reduce the chlorine required for bleaching have been vigorously pursued by the industry.

The prior art also includes bleaching processes using hydrogen peroxide, oxygen and ozone. These processes, however, also have various attendant drawbacks. Significant delignification and bleaching of Kraft pulps by the hydrogen peroxide-based processes is accompanied by unacceptable degradation of cellulose fibers and, further, is expensive. Oxygen and ozone bleaching processes also tend to degrade the cellulose fibers, in addition to degrading lignin. Degradation of cellulose results in lower pulp yields and low viscosity pulp, which is productive of paper with poor mechanical properties.

To overcome various drawbacks of the previously mentioned bleaching processes, the development of non-polluting enzymatic bleaching processes has been explored. Several types of microorganisms secrete enzymes which modify or degrade lignin without attacking cellulose or hemicellulose. The most widely studied class of these lignin degrading microorganisms is the white rot fungi. The best understood member of this class is Phanerochaete chrysosporium . However, direct use of these fungi to bleach pulp has drawbacks. See, e.g., Tran and Chambers, "Delignification of an Unbleached Hardwood Kraft Pulp by Phanerochaete chrysosporium ", Appl. Microbiol. Biotechnol. , 25, pp. 484-90 (1987). A major drawback is that fungal delignification is very slow, requiring at least seven days for appreciable brightening. Another problem is that the fungi also secrete enzymes which degrade cellulose, a most undesirable side effect. Finally, the process can only be carried out under the circumscribed conditions at which the fungi are viable. In an attempt to overcome some of these obstacles to practical enzymatic delignification, the use of enzyme preparations derived from the fungal cultures, rather than use of the fungi themselves, has been explored.

White rot fungi are presumed to accomplish the complete degradation of lignin to carbon dioxide and water through the concerted action of many enzymes. The mechanisms that underly this complex process are just now becoming known. It is thought that the initial reaction in enzymatic delignification occurs via a free-radical mechanism.

Several enzymes of the ligninolytic system of the white rot fungus Phanerochaete chrysosporium have been isolated and at least partially characterized. Members of a family of enzymes, termed lignin peroxidases ("LiP enzymes"), have been described by several groups and are commonly considered to be the enzymes most directly responsible for delignification. The lignin peroxidases require hydrogen peroxide for activity. Delignification activity of these enzymes is commonly measured in terms of their ability to oxidize veratryl alcohol (3,4-dimethoxybenzyl alcohol) to veratrylaldehyde (3,4-dimethoxybenzyl aldehyde). Veratryl alcohol has thus been used in the art as a lignin model compound, the oxidation of which is diagnostic of the presence of an enzyme that degrades lignin (also referred to as a "ligninase").

Another Phanerochaete chrysosporium enzyme recently suggested to participate in the delignification process is the Mn(II)-dependent peroxidase (also known as "NADH-oxidizing peroxidase"), hereafter referred to as "MnP". Like the LiP enzymes, MnP requires hydrogen peroxide, but, as its name suggests, it apparently also requires Mn(II). MnP does not oxidize the lignin model compound veratryl alcohol. However, it will oxidize several dyes, including 2,2′-azino-bis(3-ethyl-6-benzothiazolinesulfonate) ("ABTS") (Glenn and Gold, "Purification And Characterization of An Extracellular Mn(II)-Dependent Peroxidase From The Lignin-Degrading Basidiomycete, Phanerochaete chrysosporium ", Arch. Biochem. Biophys ., 242(2), pp. 329-41 (1985)) and phenol red. The oxidation of phenol red is a commonly used measure of MnP activity (Kuwahara et al., "Separation And Characterization Of Two Extracellular $H_2O_2$-Dependent Oxidases From Ligninoltyic Cultures Of Phanerochaete chrososporium ", FEBS Lett. , 169(2), pp. 247-50 (1984)).

The "true" function of MnP in the ligninolytic system, if any, is presently unclear. MnP has, however, recently been hypothesized to participate in the ligninolytic process by generating the hydrogen peroxide required by the LiP enzymes. This hypothesis was prompted, in part, by the recent finding that hydrogen peroxide is produced as a byproduct of the oxidation of NADH by MnP (Asada et al., "An Extracellular NADH-Oxidizing Peroxidase Produced By A Lignin-Degrading Basidiomycete, Phanerochaete chrysosporium ", J. Ferment. Technol. , 65(4), pp. 483-87 (1987)).

There is confusion in the art as to whether enzyme preparations derived from Phanerochaete chrysosporium are effective in the modification and/or degradation of lignin in wood pulp.

Farrell et al. United States patent 4,687,741 refers to (1) ligninolytic enzymes purified from Phanerochaete chrysosporium mutant strain SC26, (2) Phanerochaete chrysosporium mutant strain SC26, and (3) a process for degrading and modifying lignin in wood pulp using these purified enzymes. Farrell United States patent 4,687,745 refers to a process for enhancing the strength properties and brightness stability of mechanical pulps using enzymes derived from Phanerochaete chrysosporium strain SC26. Farrell United States patent 4,690,895 refers to a process for the bleaching of Kraft pulp using enzymes derived from Phanerochaete chrysosporium strain SC26.

In contrast to the Farrell patents, Viikari et al., "Application of Enzymes in Bleaching", Fourth International Symposium on Wood and Pulping Chemistry , Paris, France, April 1987, Vol. 1, pp. 151-54 (" Viikari I "), states:

"[T]he ligninases of P.radiata and P. chrysosporium had no detectable effect on pine kraft pulp when compared with the reference buffer treatments. The kappa numbers and the brightnesses after the ligninase treatments were virtually the same as in the reference treatments. These figures are in accordance with recent results which have shown that the reactions of extracellular ligninases lead rather to further polymerization than to degradation of lignin...."

Viikari I , page 153, left column, top paragraph (citations omitted). Viikari I offers no suggestion as to why ligninase treatment was ineffectual.

The present invention discloses, in its first embodiment, a novel process for the enzymatic delignification of lignocellulosic materials with ligninolytic enzymes that differs substantially from the processes referred to in either the Farrell patents or Viikari I .

Consistent with reports that residual lignin of Kraft pulp is crosslinked to hemicellulose, several publications refer to treatment of wood pulp with hemicellulases (e.g., xylanase) for the purpose of delignification, brightening and/or producing pulp that is productive of paper with improved mechanical properties. Those publications suggest that hemicellulases effect delignification by degrading hemicellulose, thereby rendering extractable the cleaved hemicellulose and any lignin that is crosslinked to it. See, e.g., Viikari I ; Paice et al., "Bleaching Hardwood Kraft Pulp with Enzymes from Cloned Systems", Proceedings: 74th Annual Meeting of the Canadian Pulp & Paper Association , Montreal, Canada, January 1988, pp. A133-36; Chauvet et al., "Assistance in the Bleaching of Never-Dried Pulps by the Use of Xylanases, Consequences on Pulp Properties", Fourth International Symposium on Wood and Pulping Chemistry , Paris, France, April 1987, Vol. 2, pp. 325-27; Noé et al. , "Action of Xylanases on Chemical Pulp Fibers", J. Wood Chem. Technol. , 6, pp. 167-84 (1986); Viikari et al., "Bleaching with Enzymes", Proceedings: Third International Conference on Biotechnology in the Pulp and Paper Industry , Stockholm, Sweden, June 1986, pp. 67-69; French patent 2,557,894; United States patent 2,280,307.

As stated above, Viikari I refers to treatment of wood pulp with either hemicellulases or ligninases. In addition, Viikari I refers to subjecting both hardwood and softwood unbleached Kraft pulp to treatment with hemicellulase followed by treatment with ligninase. It reports, however, that this sequential hemicellulase-ligninase treatment does not result in significantly greater delignification than hemicellulase treatment alone.

The present invention discloses, in its second embodiment, a process for delignification of lignocellulosic material by sequential treatment with ligninases and with xylanases. Unexpectedly, and contrary to the teaching of Viikari I , the process of the present invention achieves significant delignification when compared to either ligninase or xylanase treatment alone.


## SUMMARY OF THE INVENTION


This invention concerns a practical method for the efficient delignification of lignocellulosic material with ligninolytic enzymes or with ligninolytic enzymes and xylanases.

Accordingly, it is an object of this invention to provide a process which results in delignified, brightened lignocellulosic pulp without producing the corrosive, polluting effluent stream associated with conventional chlorine bleaching stages.

Another object of this invention is to provide a process which results in bleached lignocellulosic pulp productive of paper displaying improved brightness stability as compared to that produced after conventional pulp bleaching.

A still further object of this invention is to provide a process that results in a bleached lignocellulosic pulp having high viscosity, which is productive of paper with superior strength properties.

Another object of this invention is to provide an enzymatic bleaching process wherein the effluents from each stage and washing step may be recycled for use in the washing of pulp from other stages.

An additional object of this invention is to provide a multi-stage bleaching process which includes one or more ligninolytic delignification stages and, in a second embodiment, one or more xylanase delignification stages, as well as one or more conventional bleaching stages.

These and other additional objects and advantages of the present invention, apparent from the detailed description and claims that follow, are accomplished in the first embodiment of this invention by treating lignocellulosic material with a ligninolytic enzyme preparation in the presence of a low steady-state concentration of hydrogen peroxide. In a preferred process of this embodiment, at least one of the following reagents will also be present: Mn(II); an alpha-hydroxy acid; a nonionic or zwitterionic detergent; and/or a substituted aromatic alcohol capable of serving as a substrate for ligninolytic enzymes.

In a second embodiment, the objects and advantages of the present invention are accomplished by treating lignocellulosic material, in separate incubations, with a ligninolytic enzyme preparation and with a xylanase preparation. In a preferred process of this embodiment, the incubation with ligninolytic enzymes is carried out in the presence of a low steady-state concentration of hydrogen peroxide. In a more preferred process of this embodiment, at least one of the following reagents will also be present during the incubation with ligninolytic enzymes: Mn(II); an alpha-hydroxy acid; a nonionic or zwitterionic detergent; and/or a substituted aromatic alcohol capable of serving as a substrate for ligninolytic enzymes.

In a preferred embodiment of this invention, the lignocellulosic pulp is subjected to an alkaline extraction step and then washed with water following each incubation with ligninolytic enzymes or xylanases (i.e., after each enzymatic delignification step). One step of enzymatic delignification optionally followed by accompanying extraction steps constitutes a one stage enzymatic delignification process according to this invention.

## DETAILED DESCRIPTION OF THE INVENTION

The present invention relates in a first embodiment to a novel single or plural stage process for the enzymatic delignification of lignocellulosic material with ligninolytic enzymes. In a second embodiment, the process of the present invention comprises at least one ligninolytic enzyme treatment stage and at least one xylanase treatment stage. The processes of the present invention may also result in brightening of the lignocellulosic material.

An important and unexpected aspect of the second embodiment of the present invention is that the lignocellulosic material must be treated with xylanases and with ligninolytic enzymes in separate incubations in order to achieve greater delignification than treatment with xylanases or ligninases alone. It has been determined through repeated tests that treatment of lignocellulosic material with ligninolytic enzymes and xylanases in the same incubation results in less delignification than that achieved with ligninolytic enzyme treatment alone.

As used herein, the term "enzymatic delignification stage" refers to a stage comprising the step of incubating lignocellulosic material with either ligninolytic enzymes or xylanases. An enzymatic delignification stage according to this invention may further comprise extraction and/or washing steps subsequent to the step of incubating with ligninolytic enzymes or xylanases. A "ligninolytic enzyme treatment stage" is an enzymatic delignification stage comprising the step of incubating lignocellulosic material with a ligninolytic enzyme preparation. A "xylanase treatment stage" is an enzymatic delignification stage comprising the step of incubating lignocellulosic material with a xylanase preparation.

Lignocellulosic Materials

Preferred lignocellulosic materials for use in the process of the present invention are wood pulps. By way of example, such wood pulps include those prepared by the well known sulfite, sulfate or Kraft, soda and modified sulfite processes. The process of this invention will produce significant delignification of both softwood and hardwood pulps. This invention is particularly useful for the bleaching of hardwood Kraft pulp, where the kappa number of the untreated pulp is already so low that enzymatic delignification readily causes brightening.

Significant bleaching of softwood pulp produced by extensive Kraft processing (having a pre-bleaching kappa number of 8) was also observed upon treatment with ligninolytic enzymes according to the first embodiment of this invention. However, softwood pulps produced by standard Kraft processes have a very high lignin content, with a pre-bleaching kappa number of at least 20. Since the kappa number must fall below about 8 to 12 before significant increases in brightness are observed as a result of lignin removal, the degree of delignification achieved in a one or two stage process according to the first embodiment of this invention with those high kappa number pulps has not yet been sufficient to result in significant brightening. However, a process according to the first embodiment consisting of three or more enzymatic delignification stages has achieved sufficient delignification of southern softwood Kraft pulp to result in measurable brightening.

A four stage process according to the second embodiment of this invention that comprises three ligninolytic enzyme treatment stages and one xylanase treatment stage may lead to sufficient delignification of high-kappa number (i.e., kappa number greater than 20) softwood pulp to result in significant brightening.

Applicants stress that the lignocellulosic material need not be completely delignified, or even measurably brightened, for the benefits of this invention to be realized. Partial delignification of the lignocellulosic material achieved by the process of this invention would have the beneficial effect of reducing the amount of bleaching chemicals required to completely delignify and bleach the pulp. Thus, the level of chlorinated organics in the bleach plant waste effluent stream would be reduced.

Also contemplated is the enzymatic delignification of mechanical pulps, thermomechanical pulps and chemithermomechanical pulps. The foregoing is only a partial list of the lignocellulosic materials that may be utilized to good advantage. For example, use of pulps produced by processes not now known in the art is not excluded.

In addition, since the delignification process of this invention has a beneficial effect on pulp formation, the delignification and brightening of partially pulped lignocellulosics is likewise contemplated. It is envisioned that if such a partially pulped lignocellulosic material is delignified according to this invention, it would be concomitantly reduced to a pulp suitable for direct use in papermaking.

The lignocellulosic materials delignified according to the processes of this invention may be washed, or otherwise treated, prior to enzymatic delignification with either ligninolytic enzymes or xylanases. Washing steps are particularly beneficial when the lignocellulosic material used is a chemical pulp. In the preferred embodiment of this invention, chemical pulps are extensively washed with water prior to enzyme treatment. Chemical pulps may also be washed with dilute chelators, such as ethylenediamine tetraacetic acid ("EDTA") (5 to 100 mM) or diethylenetriaminepentaacetic acid ("DTPA") (0.01 to 100 mM). The lignocellulosic material is then washed extensively with water prior to enzymatic delignification. It may also be advantageous to wash the lignocellulosic material with dilute acid, for example 10 to 200 mM acetic acid, prior to enzymatic delignification. Pulp consistency would normally range from 0.1 to 10.0% during these washing steps.

The inventors also recognize the possibility that prior processing stages may involve heating of the lignocellulosic material. The realization of this possibility may require that the lignocellulosic material be cooled prior to its addition to the delignification reaction, so as to prevent heat-inactivation of the active ligninolytic enzymes or xylanases.

Ligninolytic Enzyme Preparations and Xylanase Preparations

Ligninolytic enzyme preparations derived from white rot fungi, brown rot fungi, or dry rot fungi would be useful for the purposes of this invention. Enzymes derived from mutant strains of these naturally occurring fungi would also be useful, especially if the mutant was one selected for its increased production of the desired ligninolytic enzymes. Preferably, enzymes derived from a white rot fungus will be employed. Particularly preferred is the use of enzymes derived from Phanerochaete chrysosporium . Although any strain of this fungus may be used, examples of desirable strains include SC26 (Northern Regional Research Laboratory ("NRRL") #15978), ME-446 (American Type Culture Collection ("ATTC") #34541), and VKM-F-1767 (ATCC #24725).

Xylanase preparations derived from either fungi or bacteria are useful for the purpose of this invention. Xylanase preparations comprising an endoxylanase are preferred.

The particular microorganism used as a source of xylanase does not form a part of the present invention. Rather, any microorganism found to produce xylanases, and preferably endoxylanases, would be useful for the purpose of this invention. Many such microorganisms are known in the art. See, e.g., Bastawde, "Studies on Xylanase from Chainia Sp.", doctoral thesis (in biochemistry), University of Poona, Division of Biochemical Sciences, National Chemical Laboratory, Pure - 411008, India, pp. 9-37 (May 1987) ("the Bastawde thesis"); Dekker, "Biodegradation of the Hemicelluloses", in Biosynthesis and Biodegradation of Wood Components , Academic Press, NY, pp. 505-33 (1985).

Useful fungal species include those drawn from, inter alia, Aspergillus , Chaetomium , Sporotrichum , Sclerotium , Schizophyllum , Trichoderma , and Thermoascus . Particular fungal species believed to be useful include, inter alia, Aspergillus ochraceus , Aspergillus niger , Aspergillus awamori , Sporotrichum dimorphosporum , Schizophyllum radiatum , Trichoderma reesei , Trichoderma harzianum , and Thermoascus aurantiacus .

Useful bacterial species include those drawn from Chainia * , Streptomyces , Bacillus , and Clostridium . Particular bacterial species believed to be useful include, inter alia, Streptomyces olivochromogens , Bacillus subtilus , Bacillus stearothermophilus , Clostridium thermocellum , and Clostridium acetobutylicum . Strains that produce xylanases, but not cellulases, are preferred.

Chainia species, and those Streptomyces species formerly classified as being of the Chainia genus are especially preferred as sources of xylanase preparations.

Many strains from the genera and species recited above are available at public microorganism depositories. Useful strains (i.e., those that produce xylanases) may be identified by the simple expedient of culturing the microorganisms, collecting the extracellular culture supernatant, and screening for xylanase activity according to procedures known in the art. See, e.g., Khan et al., "Assay of Xylanase and Xylosidase Activities in Bacterial and Fungal Cultures", Enzyme Microb. Technol. , 8, pp. 373-77 (1986).

We screened sixteen strains of Streptomyces and Chainia obtained from the American Type Culture

---

* It has been proposed recently that genus Chainia become a junior synonym of the genus Streptomyces and that the species it contains be renamed accord-ingly. Goodfellow et al., "Transfer of Chainia Species to the Genus Streptomyces with Emended Description of Species", System. Appl. Microbiol. , 8, pp. 55-60 (1986). Approving this suggestion, the American Type Culture Collection has reclassified the majority of its Chainia strains to genus Streptomyces . The Northern Regional Research Laboratory apparently disagrees with that proposal; it has not reclassified its Chainia species

Collection for xylanase activity. All but three were found to produce xylanases.

The lyophilized microorganisms obtained from the ATCC were cultured in the media reccommended by the ATCC (ATCC Media #5; hereinafter "Sporulation Media") (50 ml Sporulation Media in 250 ml Erlenmeyer flasks) at 26°C on a rotary shaker set at 150 rpm. After ample biomass had accumulated (approximately 2 weeks), a 10% (5 ml) inoculum was placed in 50 ml 3x Sporulation Media (contains all solutes of Sporulation Media at three-fold higher concentration) in 250 ml Erlenmeyer flasks, and incubated for 7 days at 28-30°C on a rotary shaker set at 150 rpm. After the 7 day incubation, 10% inoculums (5 ml) from each culture were placed in 3% Xylan Fermentation Media (3% Larchwood Xylan (Sigma Chemical Co. #X3875) and 1% yeast extract (Difco) in tap water) (Set A) and in 5% Wheat Bran Media (12.5% Post Natural Bran Flakes (40% wheat bran) and 1% yeast extract (Difco) in tap water) (Set B).

The cultures in 3% Xylan Fermentation Media (Set A) were incubated in 250 ml Erlenmeyer flasks at 30°C on a rotary shaker set at 220 rpm. Aliquots (3 ml) were removed from each Set A culture on each of days 3 to 8 after inoculation.

The cultures in 5% Wheat Bran Media (Set B) were incubated 4 days in 250 ml Erhlenmeyer flasks at 28°C on a rotary shaker set at 150 rpm. On the fourth day of incubation, a 10% inoculum (5 ml) of each culture from Set B was aseptically transferred to 3% Xylan Fermentation Media and incubated in 250 ml Erlenmeyer flasks at 30°C on a rotary shaker set at 220 rpm. Aliquots were removed from each Set B culture on each of days 3 to 8 after inoculation.

The aliquots were centrifuged at 70 x $g_{av}$ for 20 minutes at room temperature and the supernatants were assayed for xylanase activity as described infra . Note that since xylanase produc tion peaks at different days in different strains, it is important to assay for activity on at least each of days 3 through 8 after inoculation.

Set B was incubated in the 5% Wheat Bran Media because that media has been said to induce xylanase production. See Srinivasan et al., "Studies on Xylan Degrading Enzymes from Chainia ", Biotechnol. Lett. , 6, pp. 715-18 (1984). We, however, observed no significant difference in xylanase activity between the duplicate cultures of Set A and those of Set B.

. Using each of the screening protocols described above, we identified ten strains as positive producers of xylanase. A strain was classified as a positive producer if on any one of days 4 to 8 it was characterized by greater than 2 U/ml of xylanase activity. The ten positive producers (and their ATCC accession numbers) are: Streptomyces sclerotialus (ATCC 15896); Streptomyces flaviscleroticus (ATCC 19347); Streptomyces fumigatiscleroticus (ATCC 19345); Streptomyces minutiscleroticus (ATCC 17757); Streptomyces niger (ATCC 17756); Streptomyces ochraceiscleroticus (ATCC 15814); Streptomyces poonensis (ATCC 15723); Streptomyces roseiscleroticus (ATCC 17755); Streptomyces sp. (ATCC 27946); and Chainia hygroatrocyanea (ATCC 43962).

In addition to the ten positive producers recited above, we identified three strains as marginal producers of xylanases. A strain was classified as a marginal producer if, on its highest-producing day of days 4 to 8, it was characterized by 1-2 U/ml of xylanase activity. The three marginal producers (and their ATCC accession numbers) are: Streptomyces olivaceiscleroticus (ATCC 15722 and 15897) and Streptomyces purpurogeniscleroticus (ATCC 19348).

Only three of the sixteen strains screened were found to be negative producers. A strain was classified as a negative producer if, on its highest-producing day of days 4 to 8, it was characterized by less than 1 U/ml of xylanase activity. The negative producers (and their ATCC accession numbers) are: Streptomyces ruber (ATCC 17754); Streptomyces violens (ATCC 15898); and Chainia yaxianesis (ATCC 43139).

Most preferred as a source of endo-xylanase is Chainia sp. [NCL 82-5-1] (ATCC 53812). Xylanase II of that strain is preferred over Xylanase I. The hemicellulases of Chainia sp. [NCL 82-5-1] are discussed in the Bastawde thesis and in Srinivasan et al., "Studies on Xylan Degrading Enzymes from Chainia ", Biotechnol. Lett. , 6, pp. 715-18 (1984). For further characterization of that strain see Srinivasan et al., "High Activity Extracellular Glucose (Xylose) Isomerase from a Chainia Species", Biotechnol. Lett. , 5, pp. 611-14 (1983); Vartak et al., "Characterization of Extracellular Substrate Specific Glucose and Xylose Isomerases of Chainia ", Biotechnol. Lett. , 6, pp. 493-94 (1984); and Pawar et al., "Purification and Characterisation of Glucose (Xylose) Isomerase from Chainia Sp. (NCL 82-5-1)", Biochem. Biophys. Res. Commun. , 155, pp. 411-17 (1988). An advantageous feature of this Chainia strain is that it does not also produce cellulases. Therefore, unfractionated extracellular culture media, or concentrates threof, may be used without engendering the deleterious effects of cellulases (i.e., reduced pulp viscosity and reduced pulp yield).

Usually, the desired ligninolytic enzymes or xylanases are secreted by the microorganism into the extracellular culture medium. The extracellular culture medium is then harvested and processed to achieve the desired degree of concentration and enzyme purification. The culturing conditions and time of harvesting will influence the total amount of enzy matic activity recovered as well as the relative proportions of each of the various xylanases or ligninolytic enzymes, if more than one is present. In addition, various strains differ in their total production of ligninolytic enzymes or xylanases and in the proportions of the various enzymes produced. Therefore, one skilled in the art would optimize enzyme production with respect to the desired final use of the enzyme preparation. Methods for initiation and growth of the fungal or microbial cultures and for the harvesting of the extracellular growth medium for optimal ligninolytic enzyme or xylanase production do not form part of this invention and may conveniently be accomplished by methods known in the art.

The harvested culture media may be used directly in the delignification process. Preferably, though, the culture media is concentrated to produce an unfractionated ligninolytic enzyme concentrate or an unfractionated xylanase concentrate for use in the delignification process of the present invention.

The desirability of using extracellular growth media, or an unfractionated concentrate thereof, will be diminished when these preparations comprise significant quantities of active cellulases or proteases. Significant quantities of active cellulases would, by their attack on cellulose, cause undesirable decreases in pulp viscosity and, if enough were present, measurably decrease pulp yield. Significant quantities of active proteases might, by their attack on other proteins, cause undesirable decreases in the activity of the ligninolytic enzymes or xylanases in the reaction mixture. The undesirable effects of contaminating cellulases or proteases may be diminished or avoided by adding inhibitors of these enzymes to the unfractionated enzyme preparation. Alternatively, the enzyme preparations may be fractionated to remove some or all of any contaminating cellulases or proteases.

Any concentration method which preserves enzymatic activity would be appropriate. Suitable concentration methods include lyophilization and evaporation under vacuum, as well as precipitation with high salt, polyethylene glycol, acetone and alcohol. If any of these concentration procedures is used, the enzymes (ligninases or xylanases) may be reconstituted in an appropriate buffer (e.g., sodium acetate, sodium tartarate, sodium dimethyl succinate or sodium succinate, at pH 3-6) to produce an unfractionated enzyme concentrate for use in the delignification reaction. Alternatively, the lyophilizate or precipitate may be added directly to the delignification reaction.

The preferred method to produce unfractionated ligninolytic enzyme concentrate is by pressure dialysis of the extracellular culture media. A typical extracellular ligninolytic enzyme concentrate contains 0.5 - 20.0 U/ml of veratryl alcohol oxidizing ("VAO") activity. One unit of VAO activity is defined, for the purposes of this invention, as that amount of enzyme which is capable of oxidizing one micromole of veratryl alcohol per minute at 25°C, under the standard assay conditions described below.

VAO activity is quantified from the change in absorbance of the assay solution at 310 nm. Three prepared reagents are used in VAO activity assays: (A) 0.25 M sodium tartarate, adjusted to pH 3.0 with $H_2SO_4$; (B) 10.0 mM veratryl alcohol (Aldrich Chemical Co., Milwaukee, WI, #D13, 3OU-O); and (C) 98 mM hydrogen peroxide (Fisher Scientific, Fairlawn, NJ, #H325-5) (prepared fresh daily). To perform a VAO activity assay, the following steps are performed: (a) 400 $\mu$l of reagent A are added to a quartz cuvette; (b) 200 $\mu$l of reagent B are added; (c) 390 $\mu$l of enzyme sample/double-distilled water are added; (d) the contents of cuvette are mixed using a cable tie; (e) 10 $\mu$l reagent C are added; (f) the contents of the cuvette are mixed immediately using a cable tie; and (g) the absorbance at 310 nm is measured for 30 seconds at 25°C. The units of VAO activity per milliliter of a test sample (U/ml) is calculated as follows, using a value of 9300 $M^{-1}$ $cm^{-1}$ as the extinction coefficient for veratryl alcohol:
$(\Delta A/min.)(9300)^{-1}(1000)$ (test sample volume (ml))$^{-1}$. For the purposes of this application, one unit of LiP equals one unit of VAO activity.

The $\Delta A_{310}$/min. is linear with respect to LiP concentration within the range of 0.1 to 0.5 $\Delta A_{310}$/min. Enzyme samples yielding rates outside this range are not used to calculate activity. Instead, the volume of test sample per cuvette is reduced or increased so that the measured rate falls within the linear range.

In some circumstances, it may be desirable to use partially purified subfractions of the harvested culture media. For example, use of a ligninase-containing subfraction or a xylanase-containing subfraction that does not also contain significant quantities of cellulases or proteases is preferred. Unfractionated xylanase preparations, in particular, would often be expected to comprise cellulases. Alternatively, it may be desirous to separate LiP enzymes from any MnP enzymes, or to separate various xylanases, so that these enzymatic activities may be independently used in enzyme treatments. Finally, we contemplate the use of ligninolytic enzymes and xylanases that have been purified to near homogeneity, as well as use of mixtures of purified ligninolytic enzymes and mixtures of purified xylanases.

The methods used to subfractionate harvested extracellular culture media to achieve the desired degree of enzyme purification form no part of the present invention and may be accomplished by any effective combination of methods known to the art. For example, chromatographic separation steps based on differences in charge, pI, hydrophobicity and size may be employed. Also appropriate would be various methods of selective precipitation, for example with ammonium sulfate. In addition, we anticipate the usefulness of affinity chromatography to purify selected enzymes. Appropriate affinity ligands would include, inter alia, lectins, model lignin compounds, and monoclonal antibodies directed to the desired enzyme. See, e.g., Tan et al., United States patent 4,725,544, which describes a process for separating xylanases from mixtures thereof with other hemicellulases and cellulases produced by culturing hemicellulytic microorganisms.

An important feature of the present invention is that the purified MnP of Phanerochaete chrysosporium is effective in the delignification of wood pulp using the process of this invention. Based on the prior art (see Background Of The Invention, supra ), it was totally unexpected that MnP would be useful in the delignification and bleaching of wood pulp. As used in this application, the term "MnP" will apply to any manganese dependent peroxidase that is capable of modifying or degrading lignin, regardless of whether the enzyme is presently known in the art. As used herein, the term "ligninolytic enzymes" or "ligninases" refers to LiP and/or MnP enzymes.

Recombinant or synthetic xylanases or ligninolytic enzymes, having either LiP or MnP activity, would also be useful in the process of this invention. Such enzymes may be produced by methods known in the art.

Normally, the desired enzyme preparation is added directly to the delignification reaction. However, it may be advantageous to immobilize the enzymes on a solid support, and then add this derivatized solid support to the delignification reaction. The procedure used to immobilize will depend upon the support used, and may be accomplished by any appropriate method which does not destroy enzymatic activity. An advantage to using ligninolytic enzymes or xylanases coupled to solid supports is that the enzymes may be more conveniently recovered from the delignification reaction mixture after the desired degree of delignification has been achieved. The recovered enzyme would then be added to lignocellulosic material to initiate another delignification reaction.

Ligninolytic Enzyme Treatment Step

The step of ligninolytic enzyme treatment can be conducted in any container of the desired size for which some provision has been made for the mixing, oxygenation and temperature regulation of the contents. In addition, convenient mechanisms for the introduction of non-gaseous reaction components and the removal of reaction products must be provided. The order of addition of reaction components is not critical; however, it is preferred to add the enzymes last. The basic reaction mixture comprises the lignocellulosic material to be delignified, an active ligninolytic enzyme preparation, and hydrogen peroxide, preferably at a low steady-state concentration, all in an aqueous solution maintained at the appropriate pH. Preferably, the reaction mixture will also contain one or more of the following components: (a) a nonionic or zwitterionic detergent, (b) Mn(II), (c) an alpha-hydroxy acid, and/or (d) a substituted aromatic alcohol capable of serving as a substrate for ligninolytic enzymes.

If the lignocellulosic material to be bleached is wood pulp, it should be present in the reaction mixture at a consistency (grams dry weight pulp per gram wet weight pulp) of 0.1 to 15% and preferably 2.0 to 10%.

If a ligninolytic enzyme preparation comprising at least one LiP and devoid of any MnP activity is used, it should be present in the reaction mixture at a ratio of 10 to 100 units of VAO activity per gram dry weight pulp, and preferably 15 to 30 U/g. If an enzyme preparation comprising at least one MnP and devoid of LiP activity is used, it should be present in the reaction mixture at a ratio of 10 to 200 units per gram dry weight pulp, and preferably 35 to 100 U/g, of phenol red oxidizing ("PRO") activity.

One unit of PRO activity is defined as that amount of enzyme which causes a change in the absorbance of the assay solution, at 530 nm, of one absorbance unit per minute, at 25° C under standard assay conditions. Three prepared reagents are used in PRO activity assays. Reagent A consists of 0.11 g/L of the sodium salt of phenol red (Sigma Chemical Co., St. Louis, MO, #P-5530), 1.1 g/L of ovalbumin, and 2.5 ml/L of 85% lactic acid, in 20 mM sodium succinate, pH 4.5. Reagent B is 10 mM manganese sulfate in 20 mM sodium succinate, pH 4.5. Reagent C is 9.8 mM hydrogen peroxide (Fisher Chemical Co.) (prepared fresh daily). To perform a PRO activity assay, the following steps are carried out: (a) 900 $\mu$l of reagent A are added to a quartz cuvette; (b) 100 $\mu$l of enzyme sample/double-distilled water are added; (c) 10 $\mu$l of reagent B are added; (d) the contents of the cuvette are mixed immediately using a cable tie; (e) the spectrophotometer is blanked at 530 nm on this cuvette; (f) 10 $\mu$l reagent C are added; (g) the contents of the cuvette are mixed immediately with a cable tie; and (h) the absorbance at 530 nm is recorded for 30 seconds at 25° C. The units of PRO activity per milliliter of a test sample (U/ml) would then be calculated using the following equation: $(\Delta A/min.)(\text{test sample volume in assay } (ml))^{-1}$. For the purposes of this application, one unit of MnP equals one unit of PRO activity.

The $\Delta A_{530}/min.$ is linear with respect to MnP concentration within the range of 0.05 to 0.20 $\Delta A_{530}/min.$ Enzyme samples yielding rates outside this range are not used to calculate activity. Instead, the volume of the test sample per cuvette is reduced or increased so that the measured rate falls within the linear range.

If a ligninolytic enzyme preparation comprising one or more LiP enzymes and one or more MnP enzymes is used, they should be present in the reaction mixture at the concentrations specified above.

In the most preferred embodiment of the present invention the concentration of hydrogen peroxide is maintained at a low steady-state concentration in the reaction mixture throughout incubation with ligninolytic enzymes. The hydrogen peroxide concentration should be maintained at 0.001 to 0.5 mM. Preferably, the hydrogen peroxide concentration is maintained at 0.005 to 0.1 mM. The hydrogen peroxide concentration may be maintained conveniently at the desired level by in situ enzymatic generation, for example, through the action of glucose oxidase on glucose. Accordingly, addition to the reaction mixture of 0.001 to 10.0 U/ml glucose oxidase and 0.01 to 20.0 mM glucose would be usual. Most typically, 1.0 U/ml glucose oxidase and 0.1 to 10.0 mM glucose would be used. Alternatively, hydrogen peroxide may be supplied via continuous metered addition. Metered addition would be the preferred method of maintaining hydrogen peroxide concentration in large-scale delignification reactions. The hydrogen peroxide concentration may also be roughly maintained by periodic addition.

The pH should be maintained at 2 to 8 throughout the delignification reaction. Preferably, the pH is maintained within the range of about 3 to 5. The pH may be maintained by chemostating -- the metered or periodic addition of appropriate quantities of acid or base. Chemostating is the preferred method of pH maintenance in large-scale reactions. Alternatively, the pH may be maintained by use of a buffer in the reaction mixture. Any convenient buffer that is effective at the desired pH may be utilized. Examples of buffers appropriate at the preferred pH range include acetate, dimethyl succinate, tartarate and trans-aconitic acid. Buffer is usually added to the reaction mixture at a concentration of about 5 to 50 mM, and preferably at 15 to 25 mM.

Addition of a nonionic or zwitterionic detergent to the ligninolytic reaction mixture is preferred. Most usually, a nonionic detergent is used. Examples of appropriate nonionic detergents include octyl glucoside, polyoxyethylene glycol, and compounds from the Triton and Tween series of detergents. Tween 80 is preferred. Detergent may be added at a concentration of 0.001 to 0.1% v/v, and preferably at 0.01 to 0.05% v/v.

If the ligninolytic enzyme preparation utilized comprises an MnP, Mn(II) is required in the reaction mixture. When the ligninolytic enzyme preparation used comprises at least one LiP and excludes any MnP, the addition of Mn(II) is not essential. Mn(II) may conveniently be added as a salt, for example, as manganese sulfate or manganese acetate. Normally, Mn(II) is added as manganese sulfate. When added, Mn(II) should be present in the reaction mixture at a concentration of 0.05 to 1.0 mM. Preferably, the Mn(II) concentration will be 0.10 to 0.50 mM.

When Mn(II) is added to the reaction mixture, it is preferable to add at least one alpha-hydroxy acid as well. Appropriate alpha-hydroxy acids include malate, tartarate, citrate, lactate, phenyl-lactate, glycolate, 2-hydroxybutyrate, and salts thereof. Preferably, lactate is used. The alpha-hydroxy acid should be present in the reaction mixture at a concentration of 0.5 to 20.0 mM, and preferably at 1.0 to 10.0 mM.

If the ligninolytic enzyme preparation used comprises at least one LiP enzyme and excludes any MnP enzyme, it is preferable to add to the reaction mixture 0.005 to 0.60 mM of a substituted aromatic alcohol capable of serving as a substrate for ligninolytic enzymes. Most preferably, veratryl alcohol is added. If the ligninolytic enzyme preparation used comprises an LiP enzyme and an MnP enzyme, 0.005 to 0.60 mM of a substituted aromatic alcohol capable of serving as a substrate for ligninolytic enzymes (e.g., veratryl alcohol) may be added to the reaction mixture. If the ligninolytic enzyme preparation used comprises an MnP enzyme and excludes any LiP enzyme, then it is preferable not to add to the reaction mixture a substituted aromatic alcohol capable of serving as a substrate for ligninolytic enzymes (e.g., veratryl alcohol).

Oxygen is required for enzymatic delignification by ligninolytic enzymes. It is preferable to saturate the reaction mixture with oxygen before the addition of ligninolytic enzymes. Typically, after all reaction components have been added, the reaction vessel is briefly flushed with oxygen and then sealed to create an atmosphere enriched in oxygen.

The ligninolytic reaction mixture should be incubated at 15 to 55°C for 0.25 to 18 hours. Preferably, the delignification reaction is performed at 40 to 50°C for 2 to 8 hours. Most preferably, the delignification reaction is performed at 45°C.

It is desirable to provide for mixing of the reaction mixture components during the delignification reaction. Any method of mixing appropriate to the scale of the delignification reation may be used. However, vigorous mixing that would lead to foaming and concomitant denaturation of the enzymes should be avoided.

Xylanase Treatment Step

The step of xylanase treatment may be conducted in any container of the desired size for which provision has been made for mixing and temperature regulation of the contents. The order of addition of reaction components is not critical; however, it is preferred to add the xylanases last. The basic reaction mixture comprises the lignocellulosic material to be delignified and an active xylanase preparation, in an aqueous solution maintained at the appropriate pH.

If the lignocellulosic material to be delignified is a wood pulp, it should be present in the reaction mixture at a consistency of 0.1 to 15%, and preferably 2 to 10%.

The xylanase preparation is present in the reaction mixture at a ratio of 0.1 to 200 units xylanase activity per gram dry weight pulp. Preferably, the xylanase preparation is present at 0.1 to 50, and most preferably 1 to 25, units xylanase activity per gram dry weight pulp.

One unit of xylanase activity is defined as that amount of enzyme which causes the production from xylan of one micromole of xylose per minute, at 25°C under standard reaction conditions. Cleavage of xylan by xylanase produces reducing sugar moieties, which then are reacted with dinitrosalicylic acid ("DNSA") in the assay solution to produce a color change that is monitored at 540 nm.

A standard curve for for xylose concentration ($\mu$moles/ml) versus absorbance at 540 nm was generated using several dilutions of 100 mM xylose (Pfaltz and Bauer, Inc.) in 50 mM sodium acetate, pH 5.0. It was determined from the standard curve that one micromole of xylose has an absorbance at 540 nM of approximately 0.0128, under standard reaction conditions. This constant was used to calculate the xylanase activity of the sample solutions (e.g., culture supernatants) that were assayed.

The assay was linear for sample solutions with an absorbance at 540 nm of up to approximately 0.2. Sample solutions with absorbances above that value were diluted so that their absorbance was within the linear range.

For each sample solution (e.g., culture supernatant) assayed, a "reference solution" was prepared which contained the same amount of sample solution (e.g., culture supernatant), and enough 50 mM sodium acetate, pH 5.0 to give a final assay volume of 1 ml.

A solution of 1% larchwood xylan (Sigma Chemical Co. #X3875) in 50 mM sodium acetate, pH 5.0 (0.5 ml) was added to each sample tube. Next, a sufficient volume of 50 mM sodium acetate, pH 5.0 was added to the sample tubes to result in a final assay volume of 1 ml after the addition of sample solution (e.g., culture supernatant) and the xylan solution. Similarly, a sufficient volume of 50 mM sodium acetate was added to the reference tubes to result in a final assay volume of 1 ml after the addition of sample solution (e.g., culture supernatant). Next, the desired volume of sample solution (maximum volume, 0.5 ml) was added to each sample tube and reference tube. The tube contents were mixed gently, and incubated at 50°C for 30 minutes. After incubation, 1% DNSA in distilled water (1 ml) was added to each tube and the tubes were incubated 5 minutes at room temperature. Then, 5 N NaOH (0.2 ml) was added to each tube (to enhance color development) and the tubes were incubated for 30 minutes at room temperature. The absorbance at 540 nm of each sample solution was measured, with the spectrophotometer blanked to zero on the sample's corresponding reference solution.

The pH should be maintained at 4 to 8 throughout the incubation of xylanase with lignocellulosic material. Preferably, the pH is maintained within the range of about 5 to 7. As described above for treatment with ligninolytic enzymes, the pH may be maintained by chemostating. Chemostating is the preferred method of pH maintenance in large-scale incubations. Alternatively, the pH may be maintained by use of a buffer in the reaction mixture. Any convenient buffer that is effective at the desired pH may be utilized. Examples of appropriate buffers at the preferred pH range include acetate, tartarate, and phosphate. Buffer is usually added to the reaction mixture at a concentration of 0.1 to 100 mM, and preferably at 40 to 60 mM.

The xylanase reaction mixture should be incubated at 20 to 70°C and, preferably, at 40 to 65°C. The incubation time is 0.25 to 18 hours, preferably 0.5 to 6 hours, and most preferably 1 to 4 hours.

It is desirable to provide for mixing of the reaction components during incubation with the xylanase preparation. Any method of mixing appropriate to the scale of the incubation may be used. However, vigorous mixing that would lead to foaming and concomitant denaturation of the enzymes should be avoided.

Extraction Steps After Enzyme Treatment Step

A "one stage" enzymatic delignification process according to the first embodiment of the present invention may consist solely of the step of treating the lignocellulosic material with a ligninolytic enzyme preparation as described above. Similarly, a two stage process according to the second embodiment of the present invention may consist solely of the steps of treating the lignocellulosic material with a ligninolytic enzyme preparation, isolating the lignocellulosic material, and then treating it with a xylanase preparation (the order of the enzyme treatment steps may be reversed). Preferably, however, each stage of these processes will comprise the further step(s) of extracting the lignocellulosic material after each enzymatic delignification step.

In a preferred embodiment of this invention, the lignocellulosic material is subjected to an alkaline extraction step after each enzymatic delignification step. This alkaline extraction step involves the addition of an alkaline solution to the reaction mixture, followed by an optional incubation of up to two hours at 25 to 100°C, at a 0.1 to 10% consistency of lignocellulosic material. Preferably, the incubation will be for about 1 hour at 70°C, at a pulp consistency of 0.3 to 1.0%. After incubation the mixture is usually filtered to separate the lignocellulosic material from the alkaline solution. Any alkaline solution, such as one containing sodium hydroxide or potassium hydroxide, would be appropriate. The alkali should be present during the extraction step at a ratio of 1 to 5 grams alkali per 100 grams dry weight pulp. Preferably, the alkali will be present at a ratio of 4 grams alkali per 100 grams dry weight pulp during the alkaline extraction of softwood pulp, and at a ratio of 2 grams alkali per 100 grams dry weight pulp during the alkaline extraction of hardwood pulp.

If reuse of the enzymes in the reaction mixture is desired, they must be separated from the lignocellulosic material prior to alkaline extraction. Appropriate methods to separate the enzymes from the lignocellulosic material include vacuum filtration, precipitation and sedimentation. Normally, this separation would be accomplished by filtration. After the enzymes have been removed, the alkaline solution would be added to the lignocellulosic material and the alkaline extraction step would be carried out as described above.

The lignocellulosic material may be washed with water following the alkaline extraction step. To perform this water washing step, the lignocellulosic material is dispersed in water at a 0.1 to 10% consistency, and then collected by filtration. Preferably, this wash should be repeated one to three times. Optionally a water wash could be performed immediately after the enzymatic delignification step, with no alkaline extraction step.

Subjecting the enzyme-treated lignocellulosic material to an acid extraction step will lead to greater delignification. Acid extraction was found to produce particularly dramatic improvements in brightness of Kraft pulp which had been subjected to ligninolytic enzyme treatment in the presence of Mn(II). However, acid extraction tends not to be performed because the benefit it provides is often outweighed by its expense.

Acid extraction may be performed immediately following the alkaline extraction step. Alternatively, if the lignocellulosic material is subjected to a water washing step after the alkaline step or instead of the alkaline extraction step, the acid extraction step would be performed after that water washing step. To pezform an acid extraction step, the lignocellulosic material is collected, suspended in dilute acid, and incubated 0.1 to 10 minutes at 10 to 100°C. Preferably, the lignocellulosic material is collected by filtration, suspended in dilute acid, and incubated about 0.1 minutes at 25°C. The dilute acid solution preferably comprises 0.05 to 0.50 mM of acid. Appropriate acids include acetate, succinate, lactate, sulfurous, and other weak mineral acids. Acetic acid is preferred. The acid extraction step is performed with about 0.15 to 2.5 liters of dilute acid solution per gram dry weight of lignocellulosic material.

Plural Stage Processes

In a preferred process of the present invention, the lignocellulosic material is subjected to a plural stage enzymatic delignification process. In the first embodiment of the present invention, each stage of the plural stage process would comprise the step of treating the lignocellulosic material with a ligninolytic enzyme preparation. In the second embodiment of the present invention, at least one stage of the plural stage process would comprise the step of treating the lignocellulosic material with a xylanase preparation, and at least one other stage of the plural stage process would comprise the step of treating the lignocellulosic material with a ligninolytic enzyme preparation.

In a preferred embodiment of this invention, each enzymatic delignification stage would further comprise an alkaline extraction step, as described supra . If an alkaline extraction step is performed, each stage before the final stage will preferably further comprise a water washing step, as described supra .

The final stage may further comprise an acid extraction step, as described supra .

A plural stage process according to the first embodiment of this invention achieves greater delignification of lignocellulosic material than a one stage process, given the same total units of a ligninolytic preparation applied to the same quantity of lignocellulosic material. Each stage in a two or three stage process according to the first embodiment apparently requires fewer units of ligninolytic activity than the previous stage to achieve the same degree of delignification. One skilled in the art would accordingly apportion the ligninolytic enzyme preparation between the stages of a plural stage process according to the first embodiment. These considerations also apply to processes according to the second embodiment of this invention that comprise more than one ligninolytic enzyme treatment stage.

The second embodiment of this invention encompasses processes for delignifying lignocellulosic material comprising one or more xylanase treatment ("X") stages and one or more ligninolytic enzyme treatment ("L") stages, as described supra . The absolute number of X or L stages (so long as there be at least one of each) is not critical. At the present time, however, it appears that processes comprising more than one X stage are not justified because additional X stages have not significantly reduced lignin content. Therefore, processes according to the second embodiment of this invention comprising only one X stage are preferred. Furthermore, while any sequence order of X and L stages is permissible, placing the X stage-(s) early in the process sequence is preferred. For example, the sequence X/L is generally preferable to the sequence L/X. Similarly, the sequences X/L/L and L/X/L are generally preferable to L/L/X. Sequence order, however, has a relatively small effect on the lignin content and brightness of the treated pulp. The absolute number of L stages has a far greater impact.

Generally, the greater the number of ligninolytic enzyme treatment stages in a process according to the first or the second embodiment of the present invention, the greater the extent to which the lignocellulosic material is delignified. The preferable number of stages will be chosen by one skilled in the art based on considerations of time, economy, and compatibility with existing bleaching plant facilities. For example, a one, two, or three stage enzymatic delignification process according to the first embodiment of this invention, or a two or three stage process (comprising one xylanase treatment stage) according to the second embodiment of this invention, may be preferable to a five stage process according to either embodiment if the enzymatic delignification process is to be combined with conventional bleaching stages to produce a fully bleached product. In such a situation, the number of enzymatic bleaching stages would be chosen to optimize the economy of the entire bleaching process.

This invention also contemplates multi-stage bleaching processes of which one or more enzymatic delignification stages according to the first embodiment of this invention, or two or more stages according to the second embodiment, are but one component. The enzymatic delignification stages would be combined in series with one or more conventional bleaching stages. In such a multi-stage bleaching process, any enzymatic delignification stage that immediately precedes a conventional bleaching stage would preferably not comprise any washing or extraction steps. Examples of effective supplementary conventional bleaching steps would be stages utilizing chlorine dioxide, chlorine, hypochlorite, oxygen, ozone, hydrogen peroxide, other weak oxidants and electroreductive bleaching. Even though additional chlorine-based bleaching stages are employed in this embodiment, the total amount of chlorinated organics in the bleach plant effluent will be significantly reduced by the inclusion of one or more enzymatic delignification stages.

In order that the present invention may be more fully understood, the following examples of the process of this invention are set forth below. These examples are for purposes of illustration only and this invention should not be considered to be limited by any recitation used herein.

EXAMPLE 1

Preparation Of Unfractionated Ligninolytic Enzyme Concentrate

Ligninolytic enzyme concentrates were prepared from two strains Phanerochaete chrysosporium : VKM-F-1767 (ATTC 24725) and SC26 (NRRL 15978).

Culture strain VKM-F-1767 was initiated from spore inoculum. Spores suitable for inoculation were prepared as follows: (1) propagating the cultures on agar at 24° C for 14 to 28 days, in plates containing a spore induction medium ("Medium N" described in Vogel, "Distribution Of Lysine Pathways Among Fungi: Evolutionary Implications", Am. Nat. , XCVIII(903), pp. 435-46 (1964)), prepared with a 1/500 dilution of the Trace Element Solution, a 1/100 dilution of the Biotin Solution, without chloroform and supplemented with 2% w/v malt extract and 3% w/v yeast extract); (2) after sufficient spore development (as evidenced by the billowy appearance of the plates), washing the agar surface of each 100 x 15 mm plate with about 3 to 10 ml sterile water to liberate the spores; and (3) passing the wash water, containing spores, through a sterile funnel packed with sterile glass wool to remove contaminating fungal mycelia. The absorbance of the resulting spore suspension at 650 nm was then determined, an absorbance of 1 at 650 nm being approximately equivalent to $5 \times 10^6$ spores/ml. These spore preparations were stored at 4°C until use.

To initiate VKM-F-1767 cultures, Growth Medium A was inoculated with a spore suspension to a final concentration of at least $0.5 \times 10^5$ spores/ml, and usually to $2.5 \times 10^5$ spores/ml. Growth Medium A is the medium described in Kirk et al., "Influence Of Culture Parameters On Lignin Metabolism By Phanerochaete chrysosporium ", Arch. Microbiol. , 117, pp. 277-85 (1978), with the "Minerals" being added at a seven-fold higher concentration, and supplemented with the following reagents at the concentrations indicated: 1 mM ammonium tartarate; 0.1% v/v Tween 80; 1.8 $\mu$M manganese sulfate; 20 mM sodium acetate, pH 4.5; 6 mM benzyl alcohol; and 2% w/v glucose (as a carbon source).

Strain SC26 was initiated from starter cultures prepared by direct transfer of fungal mycelia from agar plates to 10 ml of Growth Medium A. Starter cultures were alternatively prepared by blending an existing fungal culture in a sterile Waring blender. These starter cultures were incubated at 37°C for at least three days before use.

To initiate an SC26 culture, Growth Medium A was inoculated with 1% by volume, and usually with 10% by volume, of a starter culture.

Both strains of fungi were routinely cultured in volumes of 0.1 to 1.0 liter, in 2-liter sterile containers (either Erlenmeyer flasks or roller bottles). After inoculation, the cultures were purged with oxygen and incubated at 37°C, on a rotary shaker at 50 rpm (if in an Erlenmeyer flask), or on a roller bottle incubator at 40 rpm. Mixing was commenced immediately after inoculation.

The extracellular media of the cultures was monitored for VAO activity (see Detailed Description Of The Invention, supra ) starting on the fourth day after inoculation. When at least about 0.05 U/ml of VAO activity was detected, the culture media was harvested and replaced with Growth Medium B. Growth Medium B is a low-carbon version of Growth Medium A, differing from Growth Medium A in that it contains 3.6 mM ammonium tartarate and 0.2 % w/v glucose. If an individual culture did not attain the desired level of enzymatic activity within 15 days, the media was changed to Growth Medium B in an attempt to boost enzymatic activity.

When the extracellular culture media achieved the desired level of VAO activity, it was harvested by pouring the culture through a funnel containing glass wool (thus removing fungal mycelia). Ligninolytic enzyme concentrate was then prepared from this active media by pressure dialysis using an Amicon Model 8400 ultrafiltration cell outfitted with a PM 10 dialysis membrane. Ultrafiltration was performed under 20 psi of nitrogen and was accompanied by stirring of the active media. When the volume of the media was reduced to approximately 5 to 10% of its original volume, the concentrate was poured off. Concentrate was stored at -70°C until use. This concentrated ligninolytic enzyme preparation typically contained 0.1 to 1.0 U/ml VAO activity.

### EXAMPLE 2

### Delignification And Brighteneing Of Northern Hardwood Kraft Pulp By An SC26 Enzyme Concentrate Fraction Containing PRO Activity But Substantially Devoid Of VAO Activity

In this experiment, unfractionated enzyme concentrate of Phanerochaete chrysosporium strain SC26 was fractionated by anion exchange chromatography. The fractions were assayed for VAO activity and ABTS oxidizing ("ABTSO") activity (ABTS is a peroxidase substrate that is largely resistant to LiP enzymes). The fractions' absorbance at 280 nm and 407 nm was also measured. Fractions were selected on the basis of enzymatic activity and combined to produce three pools. The fractions combined to form pool I were characterized by ABTSO activity, but exhibited no detectable VAO activity. The fractions combined to form pool III were characterized by VAO activity, but were substantially devoid of ABTSO activity. Fractions combined to form pool II exhibited both types of activity. The three pools were then assayed for their VAO, PRO, and ABTSO activity and for their absorbance at 280 nm and 407 nm. Finally, the ability of these pools to delignify and brighten hardwood Kraft pulp was tested. The surprising result was that pool I, which is totally devoid of measurable VAO activity, and thus presumably does not contain any active LiP, was very effective in pulp brightening. All steps were performed at room temperature except where otherwise noted.

Fractionation Of SC26 Enzyme Concentrate

Unfractionated enzyme concentrate was prepared from Phanerochaete chrysosporium strain SC26 as described in Example 1, with the exception that Growth Medium B was modified to replace the glucose with 0.2% w/v glycerol. A total of 1.3 L of culture supernatant (pooled from three cultures) were concentrated by pressure dialysis as described in Example 1.

The SC26 enzyme concentrate was run through a 1.5 x 6 cm column packed with Amberlite XAD-2 resin (Mallinckrodt, Inc., Paris, KY, #3409) which had been pre-equilibrated with double-distilled water. The XAD-2 column was then washed with double-distilled water, and this eluate was pooled with the flowthrough. The combined XAD-2 pool had a volume of 65 ml. It was further characterized as follows: $A_{280}$ = 2.5; $A_{407}$ = 0.5; VAO activity = 0.81 U/ml; and ABTSO activity = 9.0 U/ml.

The entire XAD-2 pool was loaded onto a 6 x 10 cm DEAE-Sephacel (Pharmacia Fine Chemicals, Uppsala, Sweden) column pre-equilibrated with double-distilled water. After loading, the column was washed with 100 ml of double-distilled water. The column was then eluted at a flowrate of about 100 -200 ml/hr with a 770-ml linear gradient of 0.1 to 0.5 M sodium chloride in 5 mM sodium tartarate, pH 4.8. Fractions (300 drops/fraction) were collected from the time the gradient was started.

Selection Of Column Fractions

Every other fraction was assayed for VAO and ABTSO activity, as well as for absorbance at 280 nm. VAO assays were performed as described in the Detailed Description Of The Invention, supra . ABTSO assays were performed as described below.

One unit of ABTSO activity was defined as that amount of enzyme which caused a change in the absorbance of the assay solution, at 415 nm, of one absorbance unit per minute, at 25°C under standard assay conditions. Three reagents were used in ABTSO assays. Reagent A consisted of 0.045 g/L of ABTS (Boehringer Mannheim, West Germany, #102946), 10 ml/L of 60% sodium lactate, 3.4 g/L bovine serum albumin or gelatin, in 50 mM sodium succinate, pH 4.5. Reagent B was 10 mM manganese sulfate in 50 mM sodium succinate, pH 4.5. Reagent C was 0.0171% hydrogen peroxide in 50 mM sodium succinate, pH 4.5 (prepared fresh daily). To perform an ABTSO assay, the following steps were carried out: a) 900 $\mu$l of reagent A were added to a quartz cuvette; b) 100 $\mu$l of test sample were added; c) 10 $\mu$l of reagent B were added; d) the contents of the cuvette were mixed immediately using a cable tie; e) the spectrophotometer was blanked at 415 nm on this cuvette; f) 10 $\mu$l of reagent C were added; g) the contents of the cuvette were mixed immediately with a cable tie; g) the absorbance at 415 nm was recorded for 30 seconds at 25°C. The units of ABTSO activity per milliliter of test sample (U/ml) were then calculated using the following equation:

($\Delta$A/min.)(test sample volume in assay (ml))$^{-1}$.

A broad, complex peak of ABTSO activity spanned approximately fractions 18 through 60. There were three discrete peaks of VAO activity spanning approximately fractions 35 to 50, 70 to 80, and 80 to 92. Column fractions were selected and combined to form three eluate pools, which were stored at -70°C until use. These pools were assayed for VAO, ABTSO, and PRO activity, as well as for their absorbance at 280 and 407 nm. The column fractions combined to form the pools, as well as the characteristics of these pools are set forth in Table I.

16

TABLE I

| Pool | I | II | III |
|------|------|------|------|
| Fractions | 18-34 | 36-48 | 72-77 & 82-89 |
| ABTSO Activity (U/ml) | 3.7 | 2.4 | 0.05 |
| PRO Activity (U/ml) | 2.9 | 0.2 | 0.03 |
| VAO Activity* (U/ml) | 0.00 ± 0.00 | 0.16 ± 0.02 | 0.42 ± 0.10 |
| $A_{280}$ | 0.43 | 0.15 | 0.20 |
| $A_{407}$ | 0.04 | 0.05 | 0.11 |

\* The values for VAO activity represent the means of two independent determinations; standard deviations are indicated. Pool I exhibited no VAO activity in either determination.

Preparation of Pulp

Thirty grams (wet weight) of northern hardwood Kraft pulp was suspended in 2 L of double-distilled water and whirled in a Waring blender (on high) for 15 seconds. The pulp was collected by vacuum filtration, resuspended in 1 L of 5 mM EDTA and collected again by vacuum filtration. The pulp was then resuspended in 1 L of 0.17 N acetic acid and collected by vacuum filtration. Finally, the pulp was resuspended in 2 L of double-distilled water, collected by vacuum filtration, and then stored at 4° C until use. This damp "washed pulp" had a consistency of 24% (0.24 g dry weight pulp per gram wet weight washed pulp).

Enzyme Reactions: Experiment A

A master mix containing the following reaction components was prepared : 175 ml of double- distilled water; 20 ml of 0.2 M sodium acetate, pH 5.0; 0.5 ml of 10% v/v Tween 80; 1.0 ml of 2.0 M lactate; 1.0 ml of 0.3 M glucose; 0.2 ml of 0.1 M manganese sulfate; 0.8 ml of 0.1 M veratryl alcohol; and 0.5 ml of NADH (2 mg/ml). Oxygen was bubbled through this master mix for three minutes. The reaction mix was then evenly distributed into ten 50-ml conical-bottom polypropylene centrifuge tubes (~20 ml per tube) and 0.5 g wet weight washed pulp (0.12 g dry weight) were added to every tube. Next, enzyme concentrate fractions were added as follows:

| Reaction | Enzyme Pool | Volume (ml) |
|----------|-------------|-------------|
| 1 | - | 0 |
| 2 | I | 1 |
| 3 | I | 2 |
| 4 | I | 4 |
| 5 | I | 10 |
| 6 | I | 20 |
| 7 | II | 2 |
| 8 | II | 5 |
| 9 | III | 2 |
| 10 | III | 5 |

Finally, 20μl of glucose oxidase (Sigma Chemical Co., #G 6500, 1.0 U/μl) were added to each tube. After adding glucose oxidase, each tube was flushed briefly with oxygen, capped, and incubated at 37° C for 18 hr, horizontally affixed to a rotary shaker set at 60 rpm.

After incubation, 0.5 N sodium hydroxide was added to each tube, to a final volume of approximately 50 ml. The contents of each tube were added to individual scintered glass filter funnels equipped with Whatman 3M filters. The empty reaction tubes were then each washed with approximately 30 ml of 0.5 N sodium hydroxide, and these washes were added to the appropriate filter funnel. The pulp in each filter funnel was collected by vacuum filtration. About 250 ml of double-distilled water was added to each filter funnel, with stirring, and the pulp was collected. Next, about 250 ml of 0.17 N acetic acid was added to each filter funnel, with stirring, and the pulp was collected again. The pulp was allowed to air-dry for three hours before measuring brightness. Brightness (% G.E.) was determined using a Technidyne Corporation Model S4 Brightimeter. A summary of the results of these reactions is presented in Table II.

TABLE II

| Reaction | VAO Activity | | PRO Activity | | Brightness |
|---|---|---|---|---|---|
| | (U/ml) | (U/g pulp) * | (U/ml) | (U/g pulp) * | (% G.E.) |
| 1 | 0.0 | 0.0 | 0.0 | 0.0 | 39.2 |
| 2 | 0.0 | 0.0 | 0.14 | 24.2 | 44.0 |
| 3 | 0.0 | 0.0 | 0.26 | 48.3 | 47.8 |
| 4 | 0.0 | 0.0 | 0.48 | 96.6 | 50.5 |
| 5 | 0.0 | 0.0 | 0.97 | 241.7 | 53.2 |
| 6 | 0.0 | 0.0 | 1.45 | 483.3 | 53.4 |
| 7 | 0.015 | 2.7 | 0.02 | 3.3 | 46.0 |
| 8 | 0.032 | 6.7 | 0.04 | 8.3 | 46.3 |
| 9 | 0.038 | 7.0 | 0.003 | 0.5 | 52.2 |
| 10 | 0.084 | 17.5 | 0.006 | 1.3 | 52.7 |

* Units per gram of dry weight pulp in the reaction mixture.

Enzyme Reactions: Experiment B

The following reagents were added to each of three 250-ml polycarbonate Erlenmeyer flasks equipped with screw caps: 125 ml of double-distilled water; 20 ml of 0.2 M sodium acetate, pH 5.0; 0.5 ml of 10% v/v Tween 80; 1.0 ml of 2.0 M lactate; 1.0 ml of 0.3 M glucose; 0.2 ml of 0.1 M manganese sulfate; 0.8 ml of 0.1 M veratryl alcohol; and 0.5 ml of NADH (2 mg/ml). Oxygen was bubbled through the reaction mixture in each flask for three minutes. Next, 5 grams of wet weight washed pulp (1.2 g dry weight) were added to each flask. Fifty milliliters of double-distilled water were added to reaction A. Fifty milliliters of pool I were added to reaction B. Fifty milliliters of pool III were added to reaction C. Finally, 0.2 ml of glucose oxidase (1.0 U/μl) were added to each flask.

After adding glucose oxidase, each flask was flushed with oxygen, capped, and incubated at 37°C for 18 hr on a rotary shaker set at 60 rpm.

After incubation, the pulp from each reaction was collected by vacuum filtration in sintered glass filter funnels equipped with Whatman 3M filters. Each pulp pad was resuspended, with stirring, in approximately 200 ml of 0.5 N sodium hydroxide, and then collected by filtration. This sodium hydroxide wash was then repeated. Finally, the pulps were resuspended, with stirring, in about 250 ml of 0.17 N acetic acid, and collected by filtration. The pulp pads were air dried at least four hours before measuring lignin content (kappa number) and brightness (% G.E.) Microkappa number was determined essentially as described in V. Berzins, "A Rapid Procedure For The Determination Of Kappa Number", Tappi , 48(1), pp. 15-18 (1965). The results of these analyses are presented in Table III.

18

## TABLE III

| Reaction | A | B | C |
|---|---|---|---|
| VAO Activity | | | |
| (U/ml) | 0.0 | 0.0 | 0.11 |
| (U/g pulp)* | 0.0 | 0.0 | 17.5 |
| PRO Activity | | | |
| (U/ml) | 0.0 | 0.73 | 0.01 |
| (U/g pulp)* | 0.0 | 120.8 | 1.3 |
| Brightness | | | |
| (% G.E.) | 41.1 | 54.5 | 51.0 |
| Lignin Content | | | |
| ($\mu$kappa No.)** | 7.9 ± 0.5 | 6.2 ± 1.2 | 7.0 ± 0.5 |

\* Units per gram of dry weight pulp in each reaction.
\** The values for microkappa number represent the means of two independent determinations; standard deviations are indicated.

## EXAMPLE 3

### Delignification And Brightening Of Northern Hardwood Kraft Pulp By A Plural Stage Treatment With VKM-F-1767 Unfractionated Enzyme Concentrate

In this experiment, northern hardwood Kraft pulp was subjected to a one, two, or three stage treatment with Phanerochaete chrysosporium VKM-F-1767 unfractionated enzyme concentrate. Each stage before the final stage of a two or three stage treatment consisted of three sequential steps: (1) incubating the pulp with the enzyme concentrate; (2) extracting the pulp with alkali; and (3) washing the pulp with water. The final stage of a plural stage treatment, or the single stage of a one stage treatment, comprised the fourth additional step of extracting the pulp with dilute acid. Three different concentrations of enzyme concentrate were tested at each stage. All but one reaction condition was run in duplicate. Control reactions lacking enzyme concentrate were run in triplicate for analysis at each stage.

### Preparation of Pulp

Northern hardwood Kraft pulp was prepared for enzyme treatment by "washing" with distilled water. Pulp (100-200 g wet weight) was dispersed in approximately 1 L distilled water using a British Sheet Disintegrator, for four to five minutes. The pulp was collected by vacuum filtration in a Buchner funnel equipped with Whatman 3M filter paper. The pulp was then washed with approximately 30 L of distilled water per 100 g wet weight pulp. To wash, the water was added in aliquots to the filter funnel, with stirring to disperse the pulp. The water was then removed after each addition by vacuum filtration and another aliquot of water was added. This process was repeated until the pulp had been washed with the desired volume of water.

The damp "washed" pulp had a consistency of 23.4% (0.234 gram dry weight pulp per gram wet weight pulp). This washed pulp had a brightness of 38% G.E., a $\mu$kappa number of 13.3 and a viscosity of 28.0 cp.

Preparation of Enzyme Concentrate

The VKM-F-1767 unfractionated enzyme concentrates were prepared as described in Example 1, using roller bottles and Growth Medium A, and stored at -70°C until used. The same concentrate preparation was used for all stage one reactions. A second concentrate preparation was used in stage two and stage three reactions; this concentrate was stored at 4°C between these stages. The VAO and PRO activities of the enzyme concentrates were measured immediately prior to each stage, according to the protocols described in the Detailed Description Of The Invention, supra . These activities were:

|  | VAO Activity | PRO Activity |
|---|---|---|
| Stage | (U/ml) | (U/ml) |
| 1 | 8.7 | 13.4 |
| 2 | 3.0 | 12.1 |
| 3 | 1.9 | 12.0 |

Stage One

A reaction mix containing all reaction components except pulp, enzyme concentrate and glucose oxidase was prepared. A sodium acetate, pH 4.5 stock solution was used in the preparation of the reaction mix, and the reaction mix was adjusted after preparation to pH 4.5 with 1.0 N sodium hydroxide. Reaction mix was added to each of 17 reaction tubes (50-ml conical bottom polypropylene centrifuge tubes) and 9 control reaction tubes, to give final reaction conditions (excluding any reaction components that may be contributed by the enzyme concentrate) of: 20 mM sodium acetate, pH 4.5; 0.025% Tween 80; 10 mM glucose; 0.45 mM veratryl alcohol; 10 mM lactic acid; 0.1 mM manganese sulfate. Oxygen was bubbled through the reaction mix in each tube for three minutes. Next, 1.7 g washed pulp (0.4 g dry weight pulp) were added to each tube, and the tubes were gently vortexed to disperse the pulp. Twenty microliters of glucose oxidase (1.0 U/μl, Sigma Chemical Co. #G 6500) were added to each tube. Enzyme concentrate and/or double-distilled water was added to give a final reaction volume of 20.0 ml in each tube: five tubes received 5.0 units LiP and 7.7 units MnP (samples 4-5, 13 and 21-22); six tubes received 10 units LiP and 15.4 units MnP (samples 6-7, 14-15 and 23-24); six tubes received 20 units LiP and 30.8 units MnP (samples 8-9, 16-17 and 25-26); and nine tubes served as controls (samples 1-3, 10-12 and 18-20), receiving only double-distilled water with no enzyme concentrate (see Table IV). Finally, the reaction tubes were purged briefly with oxygen, capped, and inverted gently several times to mix.

The reaction tubes were incubated overnight at 37°C in a G24 Environmental Incubator Shaker (New Brunswick Scientific Co., Inc., Edison, NJ), at approximately 125 rpm. The reaction tubes were affixed to the shaker platform in a horizontal position during the incubation.

After incubation, the pulps from each reaction were added to separate scintered glass funnels equipped with Whatman 3M filters. Next, 80 ml of 0.5 N sodium hydroxide were added to each filter funnel and the funnel contents were stirred. The pulps were then collected by vacuum filtration. The pulp in each filter funnel was resuspended, with stirring, in approximately 250 ml of distilled water and collected again by vacuum filtration. This water wash was repeated two times.

After the last water wash, the pulps from samples 1 to 9 were each resuspended, with stirring, in approximately 250 ml of 0.17 N acetic acid, and then collected by filtration. These pulp pads were air dried at least 12 hours before measuring the brightness (% G.E.), lignin content (μkappa number), and viscosity (cp) of the pulps. Percent G.E. and μkappa number were determined as described in Example 2. Viscosity was determined essentially as described in "Viscosity of Pulp: Capillary Viscometer Method", TAPPI, Test Method No. T230-os-76, Atlanta, GA (1976). Table V displays the results of these analyses.

Stage Two

The pulps from samples 10 to 26 were again placed in separate reaction tubes containing pH- adjusted, oxygenated reaction mix as described above. The tubes were gently vortexed to disperse the pulps, and 20 μl of glucose oxidase were added to each reaction tube. Enzyme concentrate and/or double-distilled water was added as follows, giving a final reaction volume of 20.0 ml in each tube: three tubes received 2.0 units LiP and 8.1 units MnP (samples 13 and 21-22); four tubes received 4.0 units LiP and 16.1 units MnP (samples 14-15 and 23-24); four tubes received 8.0 units LiP and 32.3 units MnP (samples 16-17 and 25-26); and six tubes served as controls (samples 10-12 and 18-20), receiving no enzyme concentrate (see Table IV). Finally, the reaction tubes were purged briefly with oxygen, capped, and inverted gently several times to mix.

The tubes were incubated as described above for stage one. The pulps were then removed from the tubes and separately washed once with sodium hydroxide and three times with water, as described above. After the last water wash, the pulps from samples 10 to 17 were washed with 0.17 N acetic acid, as described above. The resulting pulp pads were air dried at least 12 hours before measuring the brightness (% G.E.), lignin content (μkappa number), and viscosity (cp) of the pulps, as described above.

## Stage Three

The pulps from samples 18 to 26 were again placed in separate reaction tubes containing pH-adjusted, oxygenated reaction mix as described above. The tubes were gently vortexed to disperse the pulps, and 20 μl of glucose oxidase were added to each reaction tube. Enzyme concentrate and/or double-distilled water was added as follows, giving a final reaction volume of 20.0 ml in each tube: two tubes received 1.0 unit LiP and 6.3 units MnP (samples 21-22); two tubes received 2.0 units LiP and 12.6 units MnP (samples 23-24); two tubes received 4.0 units LiP and 25.3 units MnP (samples 25-26); and three tubes served as controls (samples 18-20), receiving no enzyme concentrate (see Table IV). Finally, the reaction tubes were purged briefly with oxygen, capped, and inverted gently several times to mix.

The tubes were incubated as described above for stage one. The pulps were then removed from the tubes and separately washed once with sodium hydroxide and three times with water, as described above. After the last water wash, the pulps from samples 18 to 26 were washed with 0.17 N acetic acid, as described above. These pulp pads were air dried at least 12 hours before measuring the brightness (% G.E.), lignin content (μkappa number), and viscosity (cp) of the pulps, as described above.

## Results

Table IV displays the units of LiP and MnP added per pulp sample at each stage. Units of LiP represent units of VAO activity, defined and measured as described in the Detailed Description of the Invention, supra . Units of MnP represent units of PRO activity, defined and measured as described in the Detailed Description of the Invention, supra . Units per gram dry weight pulp and units per reaction volume may be calculated from the values in Table IV since every reaction contained 0.4 g dry weight pulp in a volume of 20 ml.

Table V displays the results of this experiment -- brightness, lignin content and viscosity of the treated pulps. Replicates are grouped together. Samples 1 to 9 were assayed after one stage. Samples 10 to 17 were assayed after two stages. Samples 18 to 26 were assayed after three stages. Table V also displays the cumulative units of LiP and MnP applied to each pulp sample. For example, the value given for cumulative units of LiP for sample 26 (32.0) represents the sum of the units of LiP applied in stages one, two and three (20.0 + 8.0 + 4.0).

One stage treatment of hardwood Kraft pulp with VKM-F-1767 enzyme concentrate resulted in significant delignification and brightening of the pulp, compared with pulps from control samples that did not receive enzyme. The two stage treatments produced pulp with a lower lignin content and greater brightness than the one stage treatments. The three stage treatments worked even better than the two stage treatments. Decreased lignin content and increased brightness were obtained with acceptable decreases in viscosity.

TABLE IV

| Units LiP And MnP Added Per Reaction | | | | | | |
|---|---|---|---|---|---|---|
| | STAGE ONE | | | | | |
| Sample | LiP | MnP | | | | |
| 1 | 0.0 | 0.0 | | | | |
| 2 | 0.0 | 0.0 | | | | |
| 3 | 0.0 | 0.0 | | | | |
| 4 | 5.0 | 7.7 | | | | |
| 5 | 5.0 | 7.7 | | | | |
| 6 | 10.0 | 15.4 | | | | |
| 7 | 10.0 | 15.4 | | | | |
| 8 | 20.0 | 30.8 | | | | |
| 9 | 20.0 | 30.8 | | | | |
| | | | STAGE TWO | | | |
| | | | LiP | MnP | | |
| 10 | 0.0 | 0.0 | 0.0 | 0.0 | | |
| 11 | 0.0 | 0.0 | 0.0 | 0.0 | | |
| 12 | 0.0 | 0.0 | 0.0 | 0.0 | | |
| 13 | 5.0 | 7.7 | 2.0 | 8.1 | | |
| 14 | 10.0 | 15.4 | 4.0 | 16.1 | | |
| 15 | 10.0 | 15.4 | 4.0 | 16.1 | | |
| 16 | 20.0 | 30.8 | 8.0 | 32.3 | | |
| 17 | 20.0 | 30.8 | 8.0 | 32.3 | | |
| | | | | | STAGE THREE | |
| | | | | | LiP | MnP |
| 18 | 0.0 | 0.0 | 0.0 | 0.0 | 0.0 | 0.0 |
| 19 | 0.0 | 0.0 | 0.0 | 0.0 | 0.0 | 0.0 |
| 20 | 0.0 | 0.0 | 0.0 | 0.0 | 0.0 | 0.0 |
| 21 | 5.0 | 7.7 | 2.0 | 8.1 | 1.0 | 6.3 |
| 22 | 5.0 | 7.7 | 2.0 | 8.1 | 1.0 | 6.3 |
| 23 | 10.0 | 15.4 | 4.0 | 16.1 | 2.0 | 12.6 |
| 24 | 10.0 | 15.4 | 4.0 | 16.1 | 2.0 | 12.6 |
| 25 | 20.0 | 30.8 | 8.0 | 32.3 | 4.0 | 25.3 |
| 26 | 20.0 | 30.8 | 8.0 | 32.3 | 4.0 | 25.3 |

TABLE V

| | | Cumulative Units | | | | |
| | | Enzyme Added | | Brightness | Lignin Content | Viscosity |
| Sample | Stage | LiP | MnP | (% G.E.) | ($\mu$kappa No.) | (cp) |
|---|---|---|---|---|---|---|
| 1 | 1 | 0.0 | 0.0 | 46 | 12.2 | 27.3 |
| 2 | 1 | 0.0 | 0.0 | 48 | 10.6 | 28.2 |
| 3 | 1 | 0.0 | 0.0 | 48 | 11.1 | 27.6 |
| 4 | 1 | 5.0 | 7.7 | 46 | 11.2 | 26.7 |
| 5 | 1 | 5.0 | 7.7 | 52 | 8.9 | 24.9 |
| 6 | 1 | 10.0 | 15.4 | 53 | 7.9 | 24.9 |
| 7 | 1 | 10.0 | 15.4 | 53 | 8.5 | 24.7 |
| 8 | 1 | 20.0 | 30.8 | 54 | 8.0 | 24.5 |
| 9 | 1 | 20.0 | 30.8 | 55 | 5.2 | 24.6 |
| 10 | 2 | 0.0 | 0.0 | 49 | 10.7 | 26.4 |
| 11 | 2 | 0.0 | 0.0 | 49 | 11.7 | 26.9 |
| 12 | 2 | 0.0 | 0.0 | 49 | 11.2 | 25.2 |
| 13 | 2 | 7.0 | 15.8 | 68 | 8.8 | 20.4 |
| 14 | 2 | 14.0 | 31.5 | 67 | 6.1 | 20.4 |
| 15 | 2 | 14.0 | 31.5 | 66 | 6.1 | 20.2 |
| 16 | 2 | 28.0 | 63.1 | 66 | 4.5 | 19.8 |
| 17 | 2 | 28.0 | 63.1 | 67 | 5.2 | 20.3 |
| 18 | 3 | 0.0 | 0.0 | 51 | 10.4 | 27.4 |
| 19 | 3 | 0.0 | 0.0 | 50 | 25.5 | |
| 20 | 3 | 0.0 | 0.0 | 51 | 10.0 | 25.5 |
| 21 | 3 | 8.0 | 22.1 | 75 | 4.3 | 20.1 |
| 22 | 3 | 8.0 | 22.1 | 76 | 4.3 | 20.0 |
| 23 | 3 | 16.0 | 44.1 | 76 | 4.3 | 18.7 |
| 24 | 3 | 16.0 | 44.1 | 76 | 4.3 | 18.8 |
| 25 | 3 | 32.0 | 88.4 | 76 | 4.7 | 23.3 |
| 26 | 3 | 32.0 | 88.4 | 76 | 4.0 | 18.3 |
| Untreated washed pulp | | | | 38 | 13.3 | 28.0 |

EXAMPLE 4

<u>Delignification Of Southern Softwood Kraft Pulp By A One, Two, Or Three Stage Treatment With VKM-F-1767 Unfractionated Enzyme Concentrate</u>

In this experiment, southern softwood Kraft pulp was subjected to a one, two, or three stage treatment with Phanerochaete chrysosporium VKM-F-1767 unfractionated enzyme concentrate. The experiment was conducted as described in Example 3, with the modifications noted below.

<u>Preparation of Pulp</u>

Southern softwood Kraft pulp was prepared for enzyme treatment essentially as described in Example 3. The damp "washed" pulp had a consistency of 25.4%. This washed pulp had a brightness of 25% G.E. and a μkappa number of 25.0.

Preparation of Enzyme Concentrate

The VKM-F-1767 unfractionated enzyme concentrate was prepared as described in Example 1, using roller bottles and Growth Medium A and was stored at -70° C until used. The same concentrate preparation was used for all reactions. The enzyme concentrate was thawed and its VAO activity measured immediately prior to its addition to the stage one reactions; this value is listed below. The listed value for PRO activity of the enzyme concentrate used in the stage one reactions represents the average of the PRO activity of the concentrate before freezing and the PRO activity of the concentrate as measured before stage two. After removing aliquots for use in the stage one reactions, the enzyme concentrate was stored overnight at 4° C. The enzyme concentrate was assayed again the next day, for both VAO and PRO activities, prior to its addition to the stage two reactions. The enzyme concentrate was again stored at 4° C until stage three. In this experiment, stage three was initiated on the same day as stage two. Because the lapse of time between the second and third stages was relatively brief, the concentrate was not assayed a third time immediately prior to the third stage. Therefore, the units of LiP and MnP listed in Table VII for the stage three reactions were calculated using values for VAO and PRO activities from the assays performed immediately prior to stage two. The values for the VAO and PRO activities of the enzyme concentrate, used for calculation of enzyme units applied at each stage were:

|  | VAO Activity | PRO Activity |
|---|---|---|
| Stage | (U/ml) | (U/ml) |
| 1 | 12.0 | .23.8 |
| 2 | 11.2 | 22.5 |
| 3 | 11.2 | 22.5 |

Stage One

A 10x reaction mix of the following composition was prepared: 200 mM sodium acetate, pH 4.5; 0.5% Tween 80; 4.0 mM veratryl alcohol; 100 mM lactate; 1.0 mM manganese sulfate. This reaction mix was adjusted to pH 4.5 with sodium hydroxide after preparation. Reaction mix was added (2.0 ml per tube) to each of 11 reaction tubes and 6 control reaction tubes. Next, 1.6 g washed pulp (0.4 g dry weight pulp, were added to each tube. Double-distilled water was then added to each tube, in the volume necessary to give a final reaction volume of 20 ml per tube when all reaction components had been added. Glucose was added to each tube to give a final reaction concentration of 10.0 mM, excluding any glucose that may be contributed by the enzyme concentrate. The tubes were then vortexed to mix.

Oxygen was bubbled through the contents of the tubes for three minutes. Enzyme concentrate was then added; Table VI displays the units of LiP and MnP added to each reaction tube. Finally, 20 μl of glucose oxidase (1.0 U/μl) were added to each tube. The final reaction volume in each tube was 20 ml. After the addition of glucose oxidase, the tubes were capped and vortexed gently to mix the contents and disperse the pulp.

The samples were incubated overnight as described in Example 3. After incubation, the pulps from each reaction were washed with sodium hydroxide and then washed twice with water, as described in Example 3. After the second water wash, the pulps from samples 1 to 6 were washed with acetic acid, allowed to air dry overnight, and assayed for brightness, lignin content and viscosity as described in Example 3. Table VII displays the results of these analyses.

Stage Two

24

The pulps from samples 7 to 17 were subjected to another water wash with approximately 250 ml of distilled water. After this last wash, the pulps from these samples were again placed in reaction tubes and subjected to another incubation with enzyme concentrate, identically as described above for stage one. Table VI displays the units of LiP and MnP added to the. stage two reactions.

The stage two reactions were incubated for five hours, as described in Example 3. The pulps from each reaction were then washed once with sodium hydroxide and twice with water, as described for stage one. After the second water wash, the pulps from samples 7 to 11 were washed with acetic acid, allowed to air dry overnight, and assayed for brightness, lignin content and viscosity, as described in Example 3. Table VII displays the results of these analyses.

Stage Three

The pulps from samples 12 to 17 were subjected to another water wash with approximately 250 ml of distilled water. After this last wash, the pulps from samples 12 to 17 were again placed in reaction tubes and subjected to another incubation with enzyme concentrate, as described above for stage one. Table VI displays the units of LiP and MnP added to the stage three reactions.

The stage three reactions were incubated overnight, as described in Example 3. The pulps from each reaction were then washed with sodium hydroxide and water, as described for stage one. After the second water wash, the pulps from samples 12 to 17 were washed with acetic acid, allowed to air dry overnight, and assayed for brightness, lignin content and viscosity as described in Example 3. Table VII displays the results of these analyses.

Results

Table VI displays the units of LiP and MnP added. per pulp sample at each stage. As in Example 3, units of LiP or MnP per gram of dry weight pulp and units per volume of reaction mixture may be calculated from the values in Table VI since every reaction contained 0.4 g dry weight pulp in a volume of 20 ml.

Table VII displays the results of this experiment -- brightness, lignin content and viscosity of the treated pulps. Replicates are grouped together. Samples 1 to 6 were assayed after one stage. Samples 7 to 11 were assayed after two stages. Samples 12 to 17 were assayed after three stages. Table VI also displays the cumulative units of LiP and MnP applied to each pulp sample.

One stage treatment of southern softwood Kraft pulp with VKM-F-1767 enzyme concentrate resulted in significant delignification of the pulp, compared with pulps from control samples that did not receive enzyme concentrate. The two stage treatments produced pulp with a lower lignin content than the one stage treatments. The three stage treatments worked even better than the two stage treatments. Although significant delignification was achieved with one stage and two stage treatments, the pulps were not brightened, probably because the lignin content had not been decreased sufficiently to cause brightening. However, after a three stage treatment, the lignin content was decreased sufficiently and brightening was observed. Pulp viscosity was not reduced by the enzymatic treatments.

TABLE VI

| Units LiP And MnP Added Per Reaction | | | | | | |
|---|---|---|---|---|---|---|
| | STAGE ONE | | | | | |
| Sample | LiP | MnP | | | | |
| 1 | 0.0 | 0.0 | | | | |
| 2 | 0.0 | 0.0 | | | | |
| 3 | 9.6 | 19.0 | | | | |
| 4 | 9.6 | 19.0 | | | | |
| 5 | 19.2 | 38.1 | | | | |
| 6 | 19.2 | 38.1 | STAGE TWO | | | |
| | | | LiP | MnP | | |
| 7 | 0.0 | 0.0 | 0.0 | 0.0 | | |
| 8 | 0.0 | 0.0 | 0.0 | 0.0 | | |
| 9 | 9.6 | 19.0 | 4.8 | 9.6 | | |
| 10 | 9.6 | 19.0 | 4.8 | 9.6 | | |
| 11 | 19.2 | 38.1 | 9.6 | 19.3 | STAGE THREE | |
| | | | | | LiP | MnP |
| 12 | 0.0 | 0.0 | 0.0 | 0.0 | 0.0 | 0.0 |
| 13 | 0.0 | 0.0 | 0.0 | 0.0 | 0.0 | 0.0 |
| 14 | 9.6 | 19.0 | 4.8 | 9.6 | 0.6 | 1.2 |
| 15 | 9.6 | 19.0 | 4.8 | 9.6 | 0.6 | 1.2 |
| 16 | 19.2 | 38.1 | 9.6 | 19.3 | 1.3 | 2.6 |
| 17 | 19.2 | 38.1 | 9.6 | 19.3 | 1.3 | 2.6 |

## TABLE VII

| | | Cumulative Units | | | | |
|---|---|---|---|---|---|---|
| | | Enzyme Added | | Brightness | Lignin Content | Viscosity |
| Sample | Stage | LiP | MnP | (% G.E.) | (μkappa No.) | (cp) |
| 1 | 1 | 0.0 | 0.0 | 36 | 23.5 | |
| 2 | 1 | 0.0 | 0.0 | 36 | 22.4 | 14.3 |
| 3 | 1 | 9.6 | 19.0 | 28 | 19.9 | 21.4 |
| 4 | 1 | 9.6 | 19.0 | 27 | 20.1 | 19.4 |
| 5 | 1 | 19.2 | 38.1 | 28 | 17.9 | 18.3 |
| 6 | 1 | 19.2 | 38.1 | 28 | 19.4 | 19.5 |
| 7 | 1 | 0.0 | 0.0 | 35 | 23.0 | 7.7 |
| 8 | 1 | 0.0 | 0.0 | 34 | 22.3 | 12.8 |
| 9 | 1 | 14.4 | 28.6 | 31 | 16.0 | 14.3 |
| 10 | 2 | 14.4 | 28.6 | 30 | 15.0 | |
| 11 | 2 | 28.8 | 57.4 | 30 | 16.4 | 15.9 |
| 12 | 2 | 0.0 | 0.0 | 33 | 20.1 | |
| 13 | 2 | 0.0 | 0.0 | 35 | 20.4 | 11.9 |
| 14 | 2 | 15.0 | 29.8 | 35 | 12.4 | |
| 15 | 2 | 15.0 | 29.8 | 40 | 10.2 | 14.2 |
| 16 | 2 | 30.1 | 60.0 | 40 | 10.1 | 13.9 |
| 17 | 2 | 30.1 | 60.0 | 43 | 8.7 | 14.3 |
| Untreated washed pulp | | | | 25 | 25.0 | |

## EXAMPLE 5

### Xylanase Preparation from Chainia Sp. [NCL 82-5-1]

Chainia sp. [NCL 82-5-1] (ATCC 53812) was maintained on potato dextrose agar slants at 4°C. The microorganisms were transferred into a sterile culture medium containing a xylan source (e.g. 5% wheat bran or 1% xylan) and conventional nutrients (e.g., 1% yeast extract). The cultures were incubated at 30°C in shaker flasks with vigorous shaking until peak xylanase activity (as measured by the assay described, supra ) appeared (usually 3-5 days). After peak activity had been reached, the microbial cells and other solids were removed by conventional means (e.g., filtration or centrifugation) in order to obtain a clear filtrate or supernatant. The filtrate or supernatant was typically concentrated before use by ultra-filtration or by evaporation under vacuum. The yield of xylanase obtained from the cultures was approximately 10 U/ml if 5% wheat bran media was used, and approximately 25 U/ml if 1% xylan media was used.

## EXAMPLE 6

### Treatment Of Hardwood Kraft Pulp With Chainia sp. [NCL 82-5-1] Xylanase In Combination With A VKM-F-1767 Unfractionated Enzyme Concentrate: Concurrent vs. Sequential Treatment

In this experiment, northern hardwood Kraft pulp was treated with varying concentrations of an unfractionated xylanase preparation derived from Chainia sp. [NCL 82-5-1] (ATCC 53812), either alone, or in combination with an unfractionated enzyme concentrate derived from Phanerochaete chrysosporium strain VKM-F-1767 (ATCC 24725). The ligninolytic enzyme concentrate was applied either concurrently with the xylanase treatment, or subsequent to the xylanase treatment.

Preparation of Pulp

Northern hardwood Kraft pulp was prepared for enzyme treatment essentially as described in Example 3. The damp "washed pulp" was stored at 4°C until use. It had a consistency of 28% (0.28 g dry weight pulp per gram wet weight pulp).

Enzyme Preparations

The ligninolytic enzyme preparation utilized in this example was Phanerochaete chrysosporium VKM-F-1767 unfractionated ligninolytic enzyme concentrate. It was prepared as described in Example 1, using roller bottles and Growth Medium A and was stored at -70°C until use. The VAO and PRO activities of this concentrate were measured after thawing, as described supra . These activities were: 17.5 U/ml VAO activity (i.e., LiP) and 20.7 U/ml PRO activity (i.e., U/ml MnP).

The unfractionated xylanase preparation utilized in this example was derived from Chainia sp. [NCL 82-5-1] culture supernatant by evaporation under vacuum as described in Example 5. The powder had a xylanase activity of 2500 U/g and was stored at 20°C until use. Just prior to beginning this experiment, a "xylanase solution" was prepared containing 10 mg of the enzyme powder per milliliter 50 mM sodium acetate, pH 5.0 (i.e., 25 U/ml xylanase).

Enzyme Treatment Stages

Sample 1 was the minus enzyme contol for samples 2-4. Samples 2-4 were treated with varying concentrations of xylanase solution only. Sample 5 was the minus enzyme control for samples 6-11. Sample 6 was treated with ligninolytic enzyme con centrate only. Samples 7-10 were treated in one incubation with both xylanase solution and ligninolytic enzyme concentrate. Sample 11 was the minus enzyme control for samples 12-14. Samples 12-14 were treated in a first stage with varying concentrations of xylanase solution, and, in a second stage, with a single concentration of ligninolytic enzyme concentrate. Each enzyme treatment was performed on 0.3 g dry weight pulp, in a volume of approximately 20 ml. All enzyme incubations were performed in 50-ml conical-bottom polypropylene centrifuge tubes.

A. Samples 1-4

Twenty milliliters 50 mM sodium acetate, pH 5.0 and 1.07 g washed pulp (0.3 g dry weight pulp) were added to sample tubes 1-4. Next, xylanase solution (25 U/ml) was added to sample tubes 2-4 to achieve the ratio of xylanase units per gram dry weight pulp indicated in Table VIII. The tubes were then capped, vortexed gently to mix the contents, and incubated at 50°C for 6 hr, horizontally affixed to a rotary shaker set at 60 rpm.

After incubation, 0.5 N sodium hydroxide was added to each tube to a final volume of approximately 50 ml. The contents of each tube were added to individual sintered glass funnels equipped with Whatman 3M filters. The empty reaction tubes were then washed with approximately 30 ml 0.5 N sodium hydroxide, and these washes were added to the appropriate filter funnels. The pulp in each filter funnel was then collected by vacuum filtration. Next, about 250 ml distilled water was added to each filter funnel, with stirring, and the pulp was collected again. Finally, about 250 ml 0.17 N acetic acid was added to each filter funnel, with stirring, and the pulp was collected again.

The pulp pads were allowed to air-dry for at least 3 hr before measuring brightness and lignin content as described in Example 3.

B. Samples 5-9

A ligninase reaction mix of the following composition was prepared: 20 mM sodium acetate, pH 4.3; 0.025% Tween 80; 0.4 mM veratryl alcohol; 0.2 mM manganese sulfate; 10 mM lactate; 3 mM glucose. This reaction mix was adjusted to pH 4.3, and oxygen was bubbled through the solution for three minutes.

Twenty milliliters of ligninase reaction mix and 1.07 g washed pulp (0.3 g dry weight) were added to sample tubes 5-9. Next, xylanase solution (25 U/ml) and/or unfractionated ligninolytic enzyme concentrate (17.5 U/ml LiP; 20.7 U/ml MnP) was added to sample tubes 6-9 to achieve the ratio of enzyme units per gram dry weight pulp indicated in Table VIII. Finally, 20 μl of glucose oxidase (Sigma Chemical Co. #G-6500, 1.0 U/μl) was added to sample tubes 6-9. Every tube was then flushed briefly with oxygen, capped and vortexed gently to mix the contents. The tubes were incubated at 37°C for 18 hours, horizontally affixed to a rotary shaker set at 60 rpm.

After incubation, the pulp samples were processed and analysed for brightness and lignin content as described above for pulp samples 1-4.

C. Samples 10-14

Stage One

Twenty milliliters 50 mM sodium acetate, pH 5.0 and 1.07 g washed pulp (0.3 g dry weight pulp) were added to sample tubes 10-14. Next, xylanase solution (25 U/ml) was added to sample tubes 11-14 to achieve the ratio of enzyme units per gram dry weight pulp indicated in Table VIII. The tubes were then capped, vortexed gently to mix the contents and incubated at 50°C for 6 hours, horizontally affixed to a rotary shaker set at 60 rpm.

After the incubation with xylanase solution, the contents of each sample tube was added to a separate scintered glass funnel equipped with a Whatman 3M filter, and the pulp collected by vacuum filtration. The pulp in each funnel was then resuspended, with stirring, in approximately 250 ml of distilled water, and collected again by vacuum filtration. This water wash was repeated 2 times.

Stage Two

The pulps from sample tubes 10-14 were again placed in separate tubes. Next, 20 ml of the ligninase reaction mix (described above for samples 5-9) was added to each tube. Unfractionated ligninolytic enzyme concentrate (17.5 U/ml LiP; 20.7 U/ml MnP) was then added to sample tubes 11-14 as indicated in Table VIII. Finally, 20 μl of glucose oxidase (Sigma Chemical Co. #G-6500; 1.0 U/μl) was added to sample tubes 11-14. Every tube was then flushed briefly with oxygen, capped and vortexed gently to mix the contents. The tubes were incubated at 37°C for 18 hours, horizontally affixed to a rotary shaker set at 60 rpm.

After the incubation with ligninolytic enzyme concentrate, the pulp samples were processed and analysed for brightness and lignin content as described above for pulp samples 1-4.

Results

Table VIII displays the units of enzyme per gram dry weight pulp present in each enzyme treatment step. Units of LiP represent units of VAO activity, defined and measured as described in the Detailed Description of the Invention, supra . Units of MnP represent units of PRO activity, defined and measured as described in the Detailed Description of the Invention, supra . Units of xylanase are defined and measured as described in the Detailed Description of the Invention, supra . The entries under column heading "Enyzme Addition" symbolize the enzyme treatment each pulp sample received. For example: "X" refers to a treatment with xylanase solution and "10X" refers to a treatment with ten times that amount of xylanase solution; "X + L" refers to treatment in one incubation with both xylanase solution and ligninolytic enxyme concentrate; and X/L refers to treatment with xylanase solution in stage one and ligninolytic enzyme concentrate in stage two.

Table IX displays the results of this experiment -- brightness and lignin content of the treated pulp samples. Table X displays the percent brightening and percent delignification of the enzyme treated pulp

29

samples as compared to the appropriate minus enzyme controls (e.g., percent brightening of samples 6-10 is with respect to pulp sample 5).

TABLE VIII

| Units Enzyme Added Per Gram Dry Weight Pulp At Each Stage | | | | | | | | |
|---|---|---|---|---|---|---|---|---|
| | | STAGE ONE | | | | | | |
| Sample | Enzyme Addition | LiP | MnP | Xylanase | | | | |
| 1 | - | 0.0 | 0.0 | 0.0 | | | | |
| 2 | X | 0.0 | 0.0 | 16.7 | | | | |
| 3 | 2X | 0.0 | 0.0 | 33.3 | | | | |
| 4 | 10X | 0.0 | 0.0 | 166.7 | | | | |
| 5 | - | 0.0 | 0.0 | 0.0 | | | | |
| 6 | L | 48.3 | 57.3 | 0.0 | | | | |
| 6 | X+L | 48.3 | 57.3 | 16.7 | | | | |
| 7 | 2X+L | 48.3 | 57.3 | 33.3 | | | | |
| 8 | 10X+L | 48.3 | 57.3 | 166.7 | | | | |
| 9 | 10X+2L | 96.7 | 114.7 | 166.7 | | | | |
| | | | | | STAGE TWO | | | |
| | | | | | LiP | MnP | Xylanase | |
| 11 | -/- | 0.0 | 0.0 | 0.0 | 0.0 | 0.0 | 0.0 | |
| 12 | X/L | 0.0 | 0.0 | 16.7 | 48.3 | 57.3 | 0.0 | |
| 13 | 2X/L | 0.0 | 0.0 | 33.3 | 48.3 | 57.3 | 0.0 | |
| 14 | 10X/L | 0.0 | 0.0 | 166.7 | 48.3 | 57.3 | 0.0 | |

TABLE IX

| Sample | Enzyme Addition | Brightness (% G.E.) | Lignin Content ($\mu$kappa) |
|---|---|---|---|
| 1 | - | 41.9 | 12.0 |
| 2 | X | 44.0 | 10.2 |
| 3 | 2X | 46.2 | 10.0 |
| 4 | 10X | 47.2 | 9.4 |
| 5 | - | 41.9 | 11.6 |
| 6 | L | 52.5 | 8.4 |
| 7 | X+L | 47.3 | 9.6 |
| 8 | 2X+L | 45.2 | 9.6 |
| 9 | 10X+L | 44.9 | 10.6 |
| 10 | 10X+2L | 43.2 | 10.7 |
| 11 | -/- | 43.1 | 11.6 |
| 12 | X/L | 56.3 | 6.9 |
| 13 | 2X/L | 55.1 | 7.2 |
| 14 | 10X/L | 55.1 | 6.5 |

## TABLE X

| Sample | Enzyme Addition | Percent Brightening | Percent Delignification |
|---|---|---|---|
| 2 | X | 5 | 15 |
| 3 | 2X | 10 | 17 |
| 4 | 10X | 13 | 22 |
| 6 | L | 25 | 28 |
| 7 | X + L | 13 | 17 |
| 8 | 2X + L | 8 | 17 |
| 9 | 10X + L | 7 | 9 |
| 10 | 10X + 2L | 3 | 8 |
| 12 | X/L | 31 | 41 |
| 13 | 2X/L | 28 | 38 |
| 14 | 10X/L | 28 | 44 |

The data presented in Tables IX and X demonstrate that a sequential treatment of hardwood Kraft pulp with xylanase and then with ligninases results in a greater percent brightening and percent delignification than treatment with either xylanase or ligninases alone.

Unexpectedly, treatment of the pulp with xylanase and ligninolytic enzymes in the same incubation resulted in less brightening and delignification than treatment with ligninolytic enzymes alone.

## EXAMPLE 7

Sequential Treatment Of Hardwood Kraft Pulp With Chainia Sp. [NCL 82-5-1] Xylanase And VKM-F-1767 Unfractionated Enzyme Concentrate: Effect Of Number Of Stages And Sequence Order I

This experiment was designed to determine the effect of stage number on the single and plural stage treatment of hardwood Kraft pulp with xylanase alone and, sequentially, with xylanases and lignin-ases. Also studied was the effect of various orders of sequential treatment with these enzyme preparations.

Preparation of Pulp

Northern hardwood Kraft pulp was prepared for enzyme treatment as described in Example 3. The damp "washed" pulp had a consistency of 26.3% (0.263 g dry weight pulp per gram wet weight pulp).

Enzyme Prearations

The unfractionated ligninolytic enzyme concentrate utilized in this experiment was derived from Phanerochaete chrysosporium VKM-F-1767 as described in Example 6. This preparation was characterized by 12.4 U/ml VAO activity (LiP) and 31.5 U/ml PRO activity (MnP).

The unfractionated xylanase preparation utilized in this experiment (25 U/ml xylanase in 50 mM sodium acetate, pH 5.0) was derived from Chainia sp. [NCL 82-5-1] (ATCC 53812) culture supernatant, as described in Example 6.

Enzyme Treatments Stages

The sequence of enzyme treatment stages to which each pulp sample was subjected is indicated in Table XI under the heading "Sequence of Treatments." A treatment stage comprising the step of incubating the pulp with the unfractionated xylanase solution is indicated by an "X". A treatment stage comprising the step of treating the pulp with the unfractionated ligninolytic enzyme concentrate (containing both LiP and MnP), is indicated by an "L". A treatment stage in which neither ligninolytic enzymes nor xylanase is present (i.e., a minus enzyme control) is indicated by an "0". Successive treatment stages are separated by the symbol "/".

All enzyme incubations were conducted in 50-ml conical bottom polypropylene centrifuge tubes, in a volume of approximately 20 ml. Each sample tube received 1.9 g wet weight washed pulp (0.5 g dry weight). The cumulative units of enzyme per gram dry weight pulp with which each pulp sample was treated is indicated in Table XI.

Xylanase Treatment Stages

Xylanase treatment stages were conducted as follows. Twenty milliliters 50 mM sodium acetate, pH 5.0 was added to each pulp sample to be treated. Next, 0.5 ml xylanase solution (25 U/ml) was added to achieve the ratio of 25.0 units xylanase per gram dry weight pulp in the incubation. The tubes were then capped, vortexed gently to mix the contents and incubated at 50°C, horizontally affixed to a rotary shaker set at 60 rpm. Xylanase incubations conducted at stage one or stage three were for 16 hr. Xylanase incubations conducted at stage two or four were for 6 hr.

After each xylanase incubation that was to be followed with another enzyme treatment stage, the pulp was washed with distilled water. To wash, the contents of each tube were added to individual sintered glass funnels equipped with Whatman 3M filters. The empty reaction tubes were then washed with approximately 20-30 ml distilled water, and these washes were added to the appropriate filter funnels. The pulp in each filter funnel was then collected by vacuum filtration. Next, about 250 ml distilled water was added to each filter funnel, with stirring, and the pulp was collected again. This water wash was repeated 2 times. Finally, the pulps were returned to the reaction tubes for subsequent enzyme treatment.

Ligninolytic Enzyme Treatment Stages

Ligninolytic enzyme treatment stages were conducted as follows. A 10x ligninase reaction mix of the following composition was prepared and then adjusted to pH 4.5: 200 mM sodium acetate, pH 4.5; 0.25% Tween 80; 1.0 mM manganese sulfate; 100 mM lactate; 100 mM glucose.

Two milliliters of the 10x ligninase reaction mix and 18 ml distilled water were added to each pulp sample to be treated. Then, oxygen was bubbled through the mixture for three minutes. Next, 0.5 ml ligninolytic enzyme concentrate (12.4 U/ml LiP; 31.5 U/ml MnP) was added to achieve the ratio of 6.2 units LiP per gram dry weight pulp and 15.8 units MnP per gram dry weight pulp in the incubation. Finally, 20 $\mu$l of glucose oxidase (Sigma Chemical Co. #G-6500; 1.0 U/$\mu$l) was added. The tubes were then flushed briefly with oxygen, capped, vortexed gently to mix the contents, and incubated at 37°C, horizontally affixed to a rotary shaker set at 60 rpm. Incubations conducted at stage one or stage three were for 16 hr. Incubations conducted at stage two or four were for 6 hr.

After each incubation with unfractionated ligninolytic enzyme concentrate that was to be followed by another enzyme treatment stage, the pulp was extracted with alkali. Sodium hydroxide (0.5 N) was added to each tube to a final volume of approximately 50 ml. The contents of each tube were added to individual sintered glass funnels equipped with Whatman 3M filters. The empty reaction tubes were then washed with approximately 30 ml 0.5 N sodium hydroxide, and these washes were added to the appropriate filter funnels. The pulp in each filter funnel was then collected by vacuum filtration. The pulps were returned to the reaction tubes for subsequent enzyme treatment.

Minus Enzyme Controls

Pulp samples 18 and 19 were the "minus enzyme" controls. Pulp sample 18 was incubated with buffer and then washed with water, as described above for the xylanase treatment stages. This stage was repeated 3 times and the pulp was then extracted as described below. Pulp sample 19 was incubated with buffer, and then extracted, as described above for the ligninolytic enzyme treatment stages. This stage was repeated 3 times as described above, and the pulp was then extracted as described below.

32

## Pulp Extraction After Enzyme Treatment In Final Stage of Each Sequence Of Treatments

In the final enzyme treatment stage of each sequence of treatments, after the step of incubating with ligninolytic enzyme or xylanase, the pulp sample was extracted with alkali, washed with distilled water and then extracted with dilute acid. These extractions and washes were performed essentially as described in Example 6. The resulting pulp pads were allowed to air-dry for at least 3 hr before measuring brightness and lignin content as described in Example 3. The results of these analyses are presented in Table XI.

TABLE XI

| Sample | Sequence of Treatments | Cumulative Units | | | Brightness | Lignin Conent |
| | | Enzyme Per Gram Dry Weight Pulp | | | | |
| | | LiP | MnP | Xylanase | (% G.E.) | (μkappa) |
|---|---|---|---|---|---|---|
| 1 | X | 0.0 | 0.0 | 25.0 | 47 | 9.6 |
| 2 | X | 0.0 | 0.0 | 25.0 | 46 | 10.0 |
| 3 | X/X | 0.0 | 0.0 | 50.0 | 44 | 9.2 |
| 4 | X/X | 0.0 | 0.0 | 50.0 | 47 | 8.7 |
| 5 | X/L | 6.2 | 15.8 | 25.0 | 53 | 7.1 |
| 6 | X/L | 6.2 | 15.8 | 25.0 | 50 | 7.1 |
| 7 | L/X | 6.2 | 15.8 | 25.0 | 52 | 6.9 |
| 8 | L/X | 6.2 | 15.8 | 25.0 | 51 | 7.3 |
| 9 | X/X/X | 0.0 | 0.0 | 75.0 | 46 | 8.8 |
| 10 | X/X/X | 0.0 | 0.0 | 75.0 | 47 | 8.7 |
| 11 | X/X/L | 6.2 | 15.8 | 50.0 | 53 | 6.1 |
| 12 | X/X/L | 6.2 | 15.8 | 50.0 | 54 | 5.9 |
| 13 | L/L/X | 12.4 | 31.6 | 25.0 | 59 | 5.2 |
| 14 | X/X/X/L | 6.2 | 15.8 | 75.0 | 51 | 6.6 |
| 15 | X/X/X/L | 6.2 | 15.8 | 75.0 | 51 | 6.5 |
| 16 | L/L/L/X | 18.6 | 47.4 | 25.0 | 67 | 4.5 |
| 17 | L/L/L/X | 18.6 | 47.4 | 25.0 | 67 | 4.5 |
| 18 | 0/0/0/0 | 0.0 | 0.0 | 0.0 | 41 | 12.0 |
| 19 | 0/0/0/0 | 0.0 | 0.0 | 0.0 | 44 | 12.6 |

## EXAMPLE 8

## Sequential Treatment Of Hardwood Kraft Pulp With Chainia Sp. [NCL 82-5-1] Xylanase And VKM-F-1767 Unfractionated Enzyme Concentrate: Effect Of Number Of Stages And Sequence Order II

In this example, hardwood Kraft pulp was subjected to (a) one and two stage treatments with an unfractionated xylanase preparation derived from Chainia sp. [NCL 82-5-1], (b) one, two and three stage treatments with an unfractionated enzyme concentrate derived from Phanerochaete chrysosporium strain VKM-F-1767, and (c) various two, three, and four stage sequential treatments with both these enzyme preparations.

### Preparation of Pulp

Kraft pulp (~23% softwood, ~77% hardwood) was prepared for enzyme treatment by washing extensively in water. Pulp (about 100 g wet weight) was dispersed in approximately 2 liters distilled water using a British Sheet Disintegrator, for four to five minutes. The pulp was then washed with 15 liters distilled water as described in Example 3, and stored in a sealed plastic bag at 4°C until use. The damp "washed pulp" had a consistency of 27.2% (0.272 gram dry weight pulp per gram wet weight pulp). This washed pulp had a brightness of 35% G.E. and a μkappa number of 16.2.

## Enzyme Preparation

Two different unfractionated ligninolytic enzyme concentrates were used in this example. Both concentrates were prepared from Phanerochaete chrysosporium VKM-F-1767 as described in Example 6. Concentrate A was characterized by 1.17 U/ml VAO activity (LiP) and 14 U/ml PRO activity (MnP) when assayed immediately preceding the experiment presented in Table XII. Concentrate A was characterized by 1.15 U/ml LiP and 16.4 U/ml MnP when assayed immediately preceding the experiment presented in Table XIII. Concentrate B was characterized by 45.8 U/ml VAO activity (LiP) and 521.3 U/ml PRO activity (MnP).

The unfractionated xylanase preparation utilized in this example was a xylanase solution (25 U/ml in 50 mM sodium acetate, pH 5.0) prepared from Chainia sp. [NCL 82-5-1] culture supernatant as described in Example 6.

## Enzyme Treatment

The sequence of enzyme treatment stages to which each pulp sample was subjected is indicated in Tables XII and XIII, under the heading "Sequence of Treatments." The meaning of the symbols used to designate the sequence of treatment stages is set forth in Example 7.

To assess reproducibility, substantially the same experiment was conducted in substantially the same manner on different days. In addition, in both these experiments, each treatment sequence was evaluated on duplicate pulp samples. The results of these two independent experiments are presented at Tables XII and XIII, respectively.

All enzyme incubations were conducted in 50-ml conical bottom polypropylene centrifuge tubes, in a volume of 20 ml. Each sample tube received 1.47 g wet weight washed pulp (0.4 g dry weight). The cumulative units of enzyme per gram dry weight pulp with which each pulp sample was treated is indicated in Tables XII and XIII.

## Xylanase Treatment Stages

Xylanase treatment stages were conducted as follows. 50 mM sodium acetate, pH 5.0 (2.0 ml) and distilled water (16.4 ml) were added to each pulp sample to be treated. The tubes were briefly vortexed to disperse the pulp in the buffer. Xylanase solution (1.6 ml) was added to achieve a ratio of 100 units xylanase per gram dry weight pulp in a volume of 20 ml. The tubes were then capped, and incubated at 50°C for 2 hours, horizontally affixed to a rotary shaker set at 150 rpm.

After each xylanase incubation that was to be followed with another enzyme treatment stage, the pulp was extracted with approximately 20 ml 0.5 N sodium hydroxide, and then washed with three aliquots of distilled water (~250 ml each), essentially as described in Example 6. After extraction and water washing, the pulp pad was transferred to the appropriate tube containing the reaction mix for the subsequent stage.

## Ligninolytic Enzyme Treatment Stage

Ligninolytic enzyme treatment stages were conducted as follows. A 10x ligninase reaction mix of the following composition was prepared: 200 mM sodium acetate, pH 4.0; 0.1 M glucose; 0.1 M lactate; 1 mM manganese sulfate; 1% Tween 80. This reaction mix was then adjusted to pH 4.0. Reaction mix (2 ml) and the volume of double-distilled water necessary to give a final reaction volume of 20 ml was added to each pulp sample to be treated. The tubes were vortexed briefly to disperse the pulp.

Next, VKM-F-1767 unfractionated ligninolytic concentrate (A or B) was added generally to achieve the ratio of about 25 units PRO activity (MnP) per gram dry weight pulp in the incubation. In the experiment presented in Table XII, Concentrate A (1.17 U/ml LiP; 14 U/ml MnP) was used in all ligninolytic enzyme treatment stages, resulting in a ratio of 2.1 units LiP per gram dry weight pulp in those stages. In the experiment presented in Table XIII, Concentrate A (1.15 U/ml LiP; 16.4 U/ml MnP) was used in all stage one ligninolytic enzyme treatment stages, and in the stage two ligninolytic enzyme treatment stages of pulp samples 1 to 20, resulting in a ratio of 1.8 units LiP per gram dry weight pulp in those stages. The remaining ligninolytic enzyme treatment stages utilized Concentrate B (45.8 U/ml LiP; 521.3 U/ml MnP), resulting in a ratio of 2.2 units LiP per gram dry weight pulp in those stages. However, the stage two incubations of samples 21 and 22 with ligninolytic enzymes received slightly more Concentrate B, resulting in ratios in that stage of 2.47 units LiP, and 28.2 units MnP, per gram dry weight pulp. Finally, 20 $\mu$l glucose oxidase (Sigma Chemical Co. #G-6500; 1.0 U/$\mu$l) was added. The tubes were capped, vortexed gently to mix the contents, and incubated at 50°C for 2 hours, horizontally affixed to a rotary shaker set at 150 rpm.

After each incubation with ligninolytic concentrate that was to be followed with another enzyme treatment stage, the pulp was extracted with sodium hydroxide, and then washed with distilled water as described above for the xylanase treatment stages. After extraction and washing, the pulp pad was transferred to the appropriate tube containing the reaction mix for the subsequent stage.

Minus Enzyme Controls

Pulp samples 23 and 24 were the "minus enzyme controls" for both experiments presented in this example. They were subjected to a four stage mock (minus enzyme) treatment as described above for the ligninolytic enzyme treatment stages. After the mock enzyme incubation in the last stage, the pulps were extracted as described below.

Pulp Extraction After Enzyme Treatment In Final Stage Of Each Sequence Of Treatments

In the final enzyme treatment stage of each sequence of treatments, after the step of incubating with ligninolytic enzyme or xylanase, the pulp sample was extracted with approximately 20 ml sodium hydroxide, washed with one aliquot of distilled water (~250 ml), and extracted with one aliquot of 0.17 N acetic acid, essentially as described in Example 3. The resulting pulp pads were allowed to air-dry for at least 3 hr before measuring brightness, lignin content and viscosity as described in Example 3. The results of these analyses are presented in Tables XII and XIII.

## TABLE XII

| | | Cumulative Units | | | | | |
|---|---|---|---|---|---|---|---|
| | | Enzyme Per Gram Dry Weight Pulp | | | Brightness | Lignin Content | Viscosity |
| Sample | S quence Of Treatments | LiP | MnP | Xylanase | (% G.E.) | (μkappa) | (cp) |
| 1 | L | 2.1 | 25.0 | 0.0 | 41 | 11.4 | 23.0 |
| 2 | L | 2.1 | 25.0 | 0.0 | 44 | 11.7 | 24.4 |
| 3 | X | 0.0 | 0.0 | 100.0 | 44 | 13.2 | 28.4 |
| 4 | X | 0.0 | 0.0 | 100.0 | 45 | 13.9 | 28.7 |
| 5 | X/L | 2.1 | 25.0 | 100.0 | 47 | 10.2 | 23.9 |
| 6 | X/L | 2.1 | 25.0 | 100.0 | 48 | 9.3 | 24.4 |
| 7 | L/X | 2.1 | 25.0 | 100.0 | 45 | 10.6 | 24.0 |
| 8 | L/X | 2.1 | 25.0 | 100.0 | 44 | 10.5 | 27.8 |
| 9 | L/L | 4.2 | 50.0 | 0.0 | 52 | 9.5 | 21.3 |
| 10 | L/L | 4.2 | 50.0 | 0.0 | 53 | 8.7 | 21.9 |
| 11 | X/X | 0.0 | 0.0 | 200.0 | 45 | 12.2 | 26.0 |
| 12 | X/X | 0.0 | 0.0 | 200.0 | 46 | 11.8 | 29.3 |
| 13 | L/L/X | 4.2 | 50.0 | 100.0 | 54 | 7.9 | 21.3 |
| 14 | L/L/X | 4.2 | 50.0 | 100.0 | 53 | 7.8 | 22.2 |
| 15 | L/X/L | 4.2 | 50.0 | 100.0 | 53 | 8.1 | 20.9 |
| 16 | L/X/L | 4.2 | 50.0 | 100.0 | 52 | 6.3 | 19.7 |
| 17 | X/L/L | 4.2 | 50.0 | 100.0 | 59 | 5.9 | 21.4 |
| 18 | X/L/L | 4.2 | 50.0 | 100.0 | 57 | 6.0 | 21.5 |
| 19 | L/L/L | 6.3 | 75.0 | 0.0 | 61 | 7.0 | 19.1 |
| 20 | L/L/L | 6.3 | 75.0 | 0.0 | 63 | 6.0 | 20.0 |
| 21 | L/L/L/X | 6.3 | 75.0 | 100.0 | 64 | 7.6 | 22.0 |
| 22 | L/L/L/X | 6.3 | 75.0 | 100.0 | 65 | 4.3 | 20.0 |
| 23 | 0/0/0/0 | 0.0 | 0.0 | 0.0 | 46 | 13.4 | 26.4 |
| 24 | 0/0/0/0 | 0.0 | 0.0 | 0.0 | 47 | 13.5 | 27.3 |
| Untreated washed pulp | | | | | 35 | 16.2 | |

## TABLE XIII

| Sample | Sequence Of Treatments | Cumulative Units Enzyme Per Gram Dry Weight Pulp | | | Brightness | Lignin Content | Viscosity |
|---|---|---|---|---|---|---|---|
| | | LiP | MnP | Xylanase | (% G.E.) | (μkappa) | (cp) |
| 1 | L | 1.8 | 25.0 | 0.0 | 40 | 13.6 | 23.6 |
| 2 | L | 1.8 | 25.0 | 0.0 | 39 | 14.6 | 22.3 |
| 3 | X | 0.0 | 0.0 | 100.0 | 44 | 13.8 | 32.7 |
| 4 | X | 0.0 | 0.0 | 100.0 | 45 | 14.4 | 33.5 |
| 5 | X/L | 1.8 | 25.0 | 100.0 | 46 | 10.9 | 24.6 |
| 6 | X/L | 1.8 | 25.0 | 100.0 | 48 | 11.9 | 25.0 |
| 7 | L/X | 1.8 | 25.0 | 100.0 | 43 | 12.3 | 26.9 |
| 8 | L/X | 1.8 | 25.0 | 100.0 | 43 | 12.8 | 27.0 |
| 9 | L/L | 3.5 | 50.0 | 0.0 | 50 | 9.8 | 22.2 |
| 10 | L/L | 3.5 | 50.0 | 0.0 | 50 | 9.5 | 23.2 |
| 11 | X/X | 0.0 | 0.0 | 200.0 | 46 | 13.1 | 32.6 |
| 12 | X/X | 0.0 | 0.0 | 200.0 | 45 | 11.8 | 27.5 |
| 13 | L/L/X | 3.5 | 50.0 | 100.0 | 53 | 6.7 | 20.5 |
| 14 | L/L/X | 3.5 | 50.0 | 100.0 | 55 | 6.5 | 21.3 |
| 15 | L/X/L | 4.0 | 50.0 | 100.0 | 57 | 7.2 | 21.0 |
| 16 | L/X/L | 4.0 | 50.0 | 100.0 | 54 | 7.3 | 22.9 |
| 17 | X/L/L | 4.0 | 50.0 | 100.0 | 59 | 6.9 | 23.0 |
| 18 | X/L/L | 4.0 | 50.0 | 100.0 | 61 | 6.4 | 22.0 |
| 19 | L/L/L | 5.8 | 75.0 | 0.0 | 62 | 7.5 | 23.0 |
| 20 | L/L/L | 5.8 | 75.0 | 0.0 | 63 | 6.4 | 22.0 |
| 21 | L/L/L/X | 6.5 | 78.2 | 100.0 | 64 | 4.7 | 21.3 |
| 22 | L/L/L/X | 6.5 | 78.2 | 100.0 | 65 | 4.3 | |
| 23 | 0/0/0/0 | 0.0 | 0.0 | 0.0 | 46 | 13.6 | 29.0 |
| 24 | 0/0/0/0 | 0.0 | 0.0 | 0.0 | 45 | 12.6 | 27.2 |
| Untreated washed pulp | | | | | 35 | 16.2 | |

## EXAMPLE 9

### Sequential Treatment Of Softwood Kraft Pulp With Chainia Sp. [NCL 82-5-1] xylanase And VKM-F-1767 Unfractionated Enzyme Concentrate: Effect Of Number Of States And Sequence Order I

In this example, softwood Kraft pulp was subjected to (a) three and four stage treatments with an unfractionated xylanase preparation derived from Chainia sp. [NCL 82-5-1] (ATCC 53812), (b) three and four stage treatments with an unfractionated enzyme concentrate derived from from Phanerochaete chrysosporium strain VKM-F-1767, and (c) various three and four stage sequential treatments with both these enzyme preparations.

### Preparation of Pulp

Kraft softwood pulp was prepared for enzyme treatment by washing extensively in water. Pulp (about 100 g wet weight) was dispersed in approximately 2 liters distilled water using a British Sheet Disintegrator, for four to five minutes. The pulp was then washed with 15 liters distilled water as described in Example 3, and stored in a sealed plastic bag at 4°C until use. The damp "washed pulp" had a consistency of 18.57% (0.186 gram dry weight pulp per gram wet weight pulp). This washed pulp had a μkappa number of 27.4.

### Enzyme Preparation

The unfractionated ligninolytic enzyme concentrate used in this example was prepared from Phanerochaete chrysosporium VKM-F-1767 as described in Example 6. This concentrate was characterized by 1.84 U/ml VAO activity (LiP) and 39.5 U/ml PRO activity (MnP).

The unfractionated xylanase preparation utilized in this example was a xylanase solution (25 U/ml in 50 mM sodium acetate buffer, pH 5.0) prepared from Chainia sp. [NCL 82-5-1] culture supernatant as described in Example 6.

### Enzyme Treatment

The sequence of enzyme treatment stages to which each pulp sample was subjected is indicated in Table XIV, under the heading "Sequence of Treatments." The meaning of the symbols used to designate the sequence of treatment stages is set forth in Example 7. To assess reproducibility, each treatment sequence was evaluated on duplicate pulp samples. The results are presented in Table XIV.

All enzyme incubations were conducted in 50-ml conical bottom polypropylene centrifuge tubes, in a volume of 20 ml. Each sample tube received 2.15 g wet weight washed pulp (0.4 g dry weight). The cumulative units of enzyme per gram dry weight pulp with which each pulp sample was treated is indicated in Table XIV.

### Xylanase Treatment Stages

Xylanase treatment stages were conducted as follows. 500 mM sodium acetate buffer pH 5.0 (2 ml) and water (15.44 ml) were added to each pulp sample to be treated. The tubes were briefly vortexed to disperse the pulp. Xylanase solution (2.56 ml) was added to achieve a ratio of 160 units xylanase per gram dry weight pulp in a volume of 20 ml. The tubes were then capped, and incubated at 50°C for 2 hours, horizontally affixed to a rotary shaker set at 150 rpm.

After each xylanase incubation that was to be followed with another enzyme treatment stage, the pulp was extracted with approximately 20 ml 0.5 N sodium hydroxide, and then washed with three aliquots of distilled water (~250 ml each), essentially as described in Example 6. After extraction and water washing, the pulp pad was transferred to the appropriate tube containing the reaction mix for the subsequent stage.

### Ligninolytic Enzyme Treatment Stage

Ligninolytic enzyme treatment stages were conducted as follows. A 10x ligninase reaction mix of the following composition was prepared: 200 mM sodium acetate, pH 4.0; 0.1 M glucose; 0.1 M lactate; 1 mM manganese sulfate; 1% Tween 80. Reaction mix (2 ml) and the volume of double-distilled water necessary to give a final reaction volume of 20 ml was added to each pulp sample to be treated. The tubes were vortexed briefly to disperse the pulp.

Next, VKM-F-1767 unfractionated ligninolytic concentrate was added to achieve the ratio of 50 units PRO activity (MnP), and 2.3 units VAO activity (LiP), per gram dry weight pulp in the incubation. Finally, 20 μl glucose oxidase (Sigma Chemical Co. #G-6500; 1.0 U/μl) was added. The tubes were then capped, vortexed gently to mix the contents, and incubated at 50°C for 2 hours, horizontally affixed to a rotary shaker set at 150 rpm.

After each incubation with ligninolytic concentrate that was to be followed with another enzyme treatment stage, the pulp was extracted with sodium hydroxide, and then washed with distilled water as described above for the xylanase treatment stages. After extraction and washing, the pulp pad was transferred to the appropriate tube containing the reaction mix for the subsequent stage.

Minus Enzyme Controls

Pulp samples 21 and 22 were the "minus enzyme controls" for the experiment presented in this example. They were treated as ligninase controls. They were subjected to a four stage mock (minus enzyme) treatment as described above for the ligninolytic enzyme treatment stages. After the mock enzyme incubation in the last stage, the pulps were extracted as described below.

Pulp Extraction After Enzyme Treatment In Final Stage Of Each Sequence Of Treatments

In the final enzyme treatment stage of each sequence of treatments, after the step of incubating with ligninolytic enzyme or xylanase, the pulp sample was extracted with approximately 20 ml sodium hydroxide, washed with one aliquot of distilled water (~250 ml), and extracted with one aliquot of 0.17 N acetic acid, essentially as described in Example 3. The resulting pulp pads were allowed to air-dry for at least 3 hr before measuring brightness, lignin content and viscosity as described in Example 3. The results of these analyses are presented in Table XIV.

TABLE XIV

| | | Cumulative Units | | | | | |
|---|---|---|---|---|---|---|---|
| | | Enzyme Per Gram Dry Weight Pulp | | | Brightness | Lignin Content | Viscosity |
| Sample | Sequence Of Treatments | LiP | MnP | Xylanase | (% G.E.) | ($\mu$kappa) | (cP) |
| 1 | X/X/X | 0.0 | 0.0 | 480.0 | 35 | 18.9 | 26.3 |
| 2 | X/X/X | 0.0 | 0.0 | 480.0 | 33 | 18.7 | 23.8 |
| 3 | X/L/X | 2.3 | 49.4 | 320.0 | 35 | 17.5 | 24.9 |
| 4 | X/L/X | 2.3 | 49.4 | 320.0 | 31 | 17.3 | 24.4 |
| 5 | X/L/L | 4.6 | 98.8 | 160.0 | 30 | 13.6 | 23.0 |
| 6 | X/L/L | 4.6 | 98.8 | 160.0 | 34 | 13.4 | 19.9 |
| 7 | L/X/L | 4.6 | 98.8 | 160.0 | 41 | 9.7 | 19.6 |
| 8 | L/X/L | 4.6 | 98.8 | 160.0 | 41 | 10.0 | 19.5 |
| 9 | L/L/X | 4.6 | 98.8 | 160.0 | 32 | 15.8 | 19.9 |
| 10 | L/L/X | 4.6 | 98.8 | 160.0 | 30 | 15.1 | 17.6 |
| 11 | L/L/L | 6.9 | 148.2 | 0.0 | 42 | 9.6 | 19.7 |
| 12 | L/L/L | 6.9 | 148.2 | 0.0 | 39 | 10.9 | 17.6 |
| 13 | X/L/X/L | 4.6 | 98.8 | 320.0 | 35 | 12.8 | 17.7 |
| 14 | X/L/X/L | 4.6 | 98.8 | 320.0 | 36 | 12.9 | 19.4 |
| 15 | L/X/L/X | 4.6 | 98.8 | 320.0 | 40 | 9.9 | 17.8 |
| 16 | L/X/L/X | 4.6 | 98.8 | 320.0 | 38 | 9.1 | 19.5 |
| 17 | L/L/L/L | 9.2 | 197.6 | 0.0 | 52 | 7.0 | 16.2 |
| 18 | L/L/L/L | 9.2 | 197.6 | 0.0 | 53 | 8.1 | 17.6 |
| 19 | X/X/X/X | 0.0 | 0.0 | 540.0 | 32 | 18.6 | 24.8 |
| 20 | X/X/X/X | 0.0 | 0.0 | 540.0 | 37 | 19.0 | |
| 21 | 0/0/0/0 | 0.0 | 0.0 | 0.0 | 35 | 17.7 | 22.6 |
| 22 | 0/0/0/0 | 0.0 | 0.0 | 0.0 | 39 | 17.9 | 22.5 |

EXAMPLE 10

Sequential Treatment Of Softwood Kraft Pulp With Chainia Sp. [NCL 82-5-1] Xylanase And VKM-F-1767
Unfractionated Enzyme Concentrate: Effect Of Number Of Stages And Sequence Order II

In this example, softwood Kraft pulp was subjected to (a) two stage treatments with an unfractionated xylanase preparation derived from Chainia sp. [NCL 82-5-1], (b) two and three stage treatments with an unfractionated enzyme concentrate derived from Phanerochaete chrysosporium strain VKM-F-1767, and (c) various two and three stage sequential treatments with both these enzyme preparations.

### Preparation of Pulp

Softwood Kraft pulp was prepared for enzyme treatment by washing extensively in water as described in Example 9. The damp "washed pulp" had a consistency of 28.6% (0.286 grams dry weight pulp per gram wet weight pulp). This washed pulp had a kappa number of 29.6

### Enzyme Preparation

The unfractionated ligninolytic enzyme concentrate used in this example was prepared from Phanerochaete chrysosporium VKM-F-1767 as described in Example 6. The concentrate was characterized by 1.92 U/ml VAO activity (LiP) and 15.5 U/ml PRO activity (MnP).

The xylanase preparation utilized in this example was a xylanase solution (25 U/ml in 50 mM sodium acetate buffer, pH 5.0) prepared from Chainia sp. [NCL 82-5-1] culture supernatant as described in Example 6.

### Enzyme Treatment

The sequence of enzyme treatment stages to which each pulp sample was subjected is indicated in Table XV, under the heading "Sequence of Treatments." The meaning of the symbols used to designate the sequence of treatment stages is set forth in Example 7. To assess reproducibility, each treatment sequence was evaluated on duplicate pulp samples. The results are presented in Table XV.

All enzyme incubations were conducted in 50-ml conical bottom polypropylene centrifuge tubes, in a volume of 20 ml. Each sample tube received 1.49 g wet weight washed pulp (0.426 g dry weight). The cumulative units of enzyme per gram dry weight pulp with which each pulp sample was treated is indicated in Table XV.

### Xylanase Treatment Stages

Xylanase treatment stages were conducted as follows. 500 mM sodium acetate buffer, pH 5.0 (2 ml) and water (16.61 ml) were added to each pulp sample to be treated. The tubes were briefly vortexed to disperse the pulp. Xylanase solution (1.6 ml) was added to achieve a ratio of 160 units xylanase per gram dry weight pulp in a volume of 20 ml. The tubes were then capped, and incubated at 50°C for 2 hours, horizontally affixed to a rotary shaker set at 150 rpm.

After each xylanase incubation that was to be followed with another enzyme treatment stage, the pulp was extracted with approximately 20 ml 0.5 N sodium hydroxide, and then washed with three aliquots of distilled water (~250 ml each), essentially as described in Example 6. After extraction and water washing, the pulp pad was transferred to the appropriate tube containing the reaction mix for the subsequent stage.

### Ligninolytic Enzyme Treatment Stage

Ligninolytic enzyme treatment stages were conducted as follows. A 10x ligninase reaction mix of the following composition was prepared: 200 mM sodium acetate, pH 4.0; 0.1 M glucose; 0.1 M lactate; 1 mM manganese sulfate; 1% Tween 80. Reaction mix (2 ml) and the volume of double-distilled water necessary to give a final reaction volume of 20 ml was added to each pulp sample to be treated. The tubes were vortexed briefly to disperse the pulp.

Next, VKM-F-1767 unfractionated ligninolytic concentrate was added to achieve the ratio of 50 units PRO activity (MnP), and 6.16 units VAO activity (LiP), per gram dry weight pulp in the incubation. Finally, 20 $\mu$l glucose oxidase (Sigma Chemical Co. #G-6500; 1.0 U/$\mu$l) was added. The tubes were then capped, vortexed gently to mix the contents, and incubated at 50° C for 2 hours, horizontally affixed to a rotary shaker set at 150 rpm.

After each incubation with ligninolytic concentrate that was to be followed with another enzyme treatment stage, the pulp was extracted with sodium hydroxide, and then washed with distilled water as described above for the xylanase treatment stages. After extraction and washing, the pulp pad was transferred to the appropriate tube containing the reaction mix for the subsequent stage.

Minus Enzyme Controls

Pulp samples 9-10 and 11-12 were the "minus enzyme controls" for the experiment presented in this example. They were treated as ligninase controls. Samples 9-10 and 10-11 were subjected respectively to a two stage and a three stage mock (minus enzyme) treatment as described above for the ligninolytic enzyme treatment stages. After the mock enzyme incubation in the last stage, the pulps were extracted as described below.

Pulp Extraction After Enzyme Treatment In Final Stage Of Each Sequence Of Treatments

In the final enzyme treatment stage of each sequence of treatments, after the step of incubating with ligninolytic enzyme or xylanase, the pulp sample was extracted with approximately 20 ml sodium hydroxide, washed with one aliquot of distilled water (~250 ml), and extracted with one aliquot of 0.17 N acetic acid, essentially as described in Example 3. The resulting pulp pads were allowed to air-dry for at least 3 hr before measuring brightness, lignin content and viscosity as described in Example 3. The results of these analyses are presented in Table XV.

TABLE XV

| | | Cumulative Units | | | | | |
| | | Enzyme Per Gram Dry Weight Pulp | | | Brightness | Lignin Content | Viscosity |
| Sample | Sequence Of Treatments | LiP | MnP | Xylanase | (% G.E.) | ($\mu$kappa) | (cP) |
|---|---|---|---|---|---|---|---|
| 1 | X/X | 0.0 | 0.0 | 320.0 | 29 | 20.9 | 32.5 |
| 2 | X/X | 0.0 | 0.0 | 320.0 | 29 | 19.9 | 31.2 |
| 3 | L/L | 12.3 | 99.7 | 0.0 | 28 | 13.7 | 17.5 |
| 4 | L/L | 12.3 | 99.7 | 0.0 | 29 | 15.9 | 21.0 |
| 5 | X/L | 6.2 | 49.8 | 160.0 | 27 | 17.2 | 26.2 |
| 6 | X/L | 6.2 | 49.8 | 160.0 | 26 | 16.5 | 23.6 |
| 7 | L/X | 6.2 | 49.8 | 160.0 | 25 | 20.8 | 22.8 |
| 8 | L/X | 6.2 | 49.8 | 160.0 | 23 | 19.6 | 22.8 |
| 9 | 0/0 | 0.0 | 0.0 | 0.0 | 30 | 23.8 | 30.4 |
| 10 | 0/0 | 0.0 | 0.0 | 0.0 | 30 | 22.2 | 25.4 |
| 11 | 0/0/0 | 0.0 | 0.0 | 0.0 | 30 | 24.5 | 27.9 |
| 12 | 0/0/0 | 0.0 | 0.0 | 0.0 | 32 | 22.8 | 27.7 |
| 13 | X/L/L | 12.3 | 99.7 | 160.0 | 30 | 13.5 | 19.8 |
| 14 | X/L/L | 12.3 | 99.7 | 160.0 | 33 | 11.4 | 21.2 |
| 15 | L/X/L | 12.3 | 99.7 | 160.0 | 32 | 12.5 | 18.9 |
| 16 | L/X/L | 12.3 | 99.7 | 160.0 | 30 | | 23.0 |
| 17 | L/L/X | 12.3 | 99.7 | 160.0 | 27 | 14.1 | 21.8 |
| 18 | L/L/X | 12.3 | 99.7 | 160.0 | 28 | 13.0 | 22.4 |
| 19 | L/L/L | 18.5 | 149.4 | 0.0 | 31 | 13.5 | 17.5 |
| 20 | L/L/L | 18.5 | 149.4 | 0.0 | 37 | 11.1 | 18.7 |

## Claims

1. A process for delignifying lignocellulosic material, characterized by the fact that it comprises two or more enzymatic delignification stages, wherein the enzymatic delignification stages comprise:

(a) one or more ligninolytic enzyme treatment stages, wherein each ligninolytic enzyme treatment stage comprises the step of incubating the lignocellulosic material in a ligninolytic reaction mixture comprising an effective amount of a ligninolytic enzyme preparation; and

(b) one or more xylanase treatment stages, wherein each xylanase treatment stage comprises the step of incubating the lignocellulosic material in a xylanase reaction mixture comprising an effective amount of xylanase preparation.

2. The process according to claim 1, characterized by the fact that the lignocellulosic material is wood pulp.

3. The process according to claim 1, characterized by the fact that the ligninolytic enzyme preparation is derived from a white rot fungus.

4. The process according to claim 3, characterized by the fact that the white rot fungus is a strain of Phanerochaete chrysosporium .

5. The process according to claim 4, characterized by the fact that the strain of Phanerochaete chrysosporium is a member selected from the group consisting of: SC26, having the identifying characteristics of NRRL 15978, VKM-F-1767, having the identifying characteristics of ATTC 24725; and ME-446, having the identifying characteristics of ATCC 34541.

6. The process according to claim 3, characterized by the fact that the ligninolytic enzyme preparation is an unfractionated enzyme concentrate consisting of concentrated extracellular culture media of the white rot fungus.

7. The process according to claim 1, characterized by the fact that the ligninolytic enzyme preparation comprises at least one lignin peroxidase.

8. The process according to claim 1, characterized by the fact that the ligninolytic enzyme preparation comprises at least one Mn(II)-dependent peroxidase.

9. The process according to claim 1, characterized by the fact that the ligninolytic enzyme preparation comprises at least one lignin peroxidase and at least one Mn(II) dependent peroxidase.

10. The process according to claim 1, characterized by the fact that the xylanase preparation is derived from a microorganism selected from the group consisting of strains of Aspergillus , Sporotrichum , Sclerotium , Chaetomium , Schizophyllum , Chainia , Clostridium , Streptomyces , Bacillus and Trichoderma or is derived from a strain of Chainia or Streptomyces .

11. The process according to claim 10, characterized by the fact that the strain is selected from the group consisting of: Streptomyces sclerotialus , having the identifying characteristics of ATCC 15896; Streptomyces flaviscleroticus , having the identifying characteristics of ATCC 19347; Streptomyces fumigatiscleroticus , having the identifying characteristics of ATCC 19345; Streptomyces minutiscleroticus , having the identifying characteristics of ATCC 17757; Streptomyces niger , having the identifying characteristics of ATCC 17756; Streptomyces ochraceiscleroticus , having the identifying characteristics of ATCC 15814; Streptomyces poonensis , having the identifying characteristics of ATCC 15723; Streptomyces roseiscleroticus , having the identifying characteristics of ATCC 17755; Streptomyces sp. , having the identifying characteristics of ATCC 27946; and Chainia hygroatrocyanea , having the identifying characteristics of ATCC 43962.

12. The process according to claim 1, characterized by the fact tht the ligninolytic reaction mixture further comprises hydrogen peroxide, and wherein the hydrogen peroxide is maintained at a steady-state concentration of about 0.001 to 0.5 mM.

13. The process according to claim 12, characterized by the fact that the ligninolytic reaction mixture further comprises: 0.5 to 1.0 mM Mn(II).

14. The process according to claim 13, characterized by the fact that the ligninolytic reaction mixture furthr comprises: 0.5 to 20mM of an $\alpha$-hydroxy acid.

15. The process according to claim 14, characterized by the fact that the ligninolytic reaction mixture further comprises: 0.001 to 0.1% of a detergent selected from the group consisting of nonionic and zwitterionic detergents.

16. The process according to claim 12, characterized by the fact that the ligninolytic reaction mixture further comprises: 0.001 to 0.1% of a detergent selected from the group consisting of nonionic and zwitteronic detergents.

42

17. The process according to anyone of claims 12 through 16, characterized by the fact that the hydrogen peroxide concentration is maintained at the steady-state level by the in situ enzymatic generation of hydrogen peroxide.

18. The process according to claim 17, characterized by the fact that the hydrogen peroxide is generated in situ by the action of 0.001 to 10 U/ml glucose oxidase on 0.01 to 20 mM glucose.

19. The process according to anyone of claims 12 through 16, characterized by the fact that the hydrogen peroxide concentration is maintained at the steady-state level by the metered or periodic addition of hydrogen peroxide.

20. The process according to claims 13, 14 or 15, characterized by the fact that the Mn(II)is supplied as manganese sulfate.

21. The process according to claims 14 or 15, characterized by the fact that the $\alpha$-hydroxy acid is lactic acid.

22. The process according to claim 1, characterized by the fact that each of the enzymatic delignification stages comprises the further subsequent step of extracting the lignocellulosic material with alkali.

23. The process according to claim 1, characterized by the fact tht one or more of the enzymatic delignification stages comprises the further subsequent step of extensively washing the lignocellulosic material with water.

24. The process according to claim 22, characterized by the fact that each of the enzymatic delignification stages comprises the further subsequent step of extensively washing the lignocellulosic material with water.

25. The process according to claims 22, 23 or 24, characterized by the fact that the final enzyme treatment stage comprises the further subsequent step of extracting the lignocellulosic material with a dilute acid solution.

26. The process according to claim 1, characterized by the fact that

(a) the incubation with the ligninolytic enzyme preparation is performed at 15 to 55 °C for 0.25 to 18 hours, and

(b) the incubation with the xylanase preparation is performed at 20 to 70 °C for 0.25 to 18 hours.

27. The process according to claim 1, chracterized by the fact that it comprises at least one conventional bleaching and at least two enzymatic delignification stages.